# EUROPEAN PATENT APPLICATION

(11) **EP 4 134 132 A1**
(43) Date of publication of application: **15.02.2023**
(21) Application number: 21785165.8
(22) Date of filing: 05.04.2021
(51) Int. Cl.: A61P 9/00, A61P 9/10, A61P 25/00, A61P 43/00, A61P 17/02, C07K 14/515, A61K 9/06, C12M 1/00, C12M 3/00, C12N 5/071, C12N 5/0797, A61K 47/42, A61K 38/02, A61K 38/18, A61K 35/12, A61K 35/30, A61K 35/51, A61L 27/36, A61K 31/737

(54) **UNIT FOR ANGIOGENESIS PROMOTION AND/OR NERVE REGENERATION**

(30) Priority: 08.04.2020 JP 2020069686; 15.05.2020 JP 2020085736; 28.08.2020 JP 2020144278; 29.01.2021 JP 2021013172
(71) Applicant: Ichimaru Pharcos Co., Ltd., Motosu-shi, Gifu 501-0475 (JP); Kobe Gakuin Educational Foundation, Kobe-shi, Hyogo 650-8586 (JP)
(72) Inventor: MASUTANI Teruaki, Motosu-shi, Gifu 501-0475 (JP); MIZUTANI Kenichi, Kobe-shi, Hyogo 650-8586 (JP)
(74) Representative: Winter, Brandl - Partnerschaft mbB
(86) International application number: PCT/JP2021/014496
(87) International publication number: WO 2021/206055

(57) **Abstract**

There is a need to develop means for safe angiogenesis promotion and/or nerve regeneration (e.g., technology to induce angiogenesis in cells and tissues transplanted into the body), and agents such as scaffolds for neural stem cells (undifferentiated tissue stem cells of the nervous system) to be viable and proliferate after such transplantation, and to provide such means.

Provided is a unit used to promote angiogenesis and/or nerve regeneration, including a gel component and proteoglycans, and the like.

## Description

### Cross reference

This application claims priority based on Japanese Patent Applications No. 2020-069686 filed in Japan on April 8, 2020, No. 2020-085736 filed on May 15, 2020, No. 2020-144278 filed on August 28, 2020,and No. 2021-013172 filed on January 29, 2021, the entire contents of which are incorporated herein by reference in their entirety. In addition, all the contents described in all patents, patent applications, and documents cited in the present application are incorporated herein by reference in their entirety.

### Technical Field

The present invention relates to a unit for promoting angiogenesis and/or nerve regeneration in humans and the like.

### Background Art

Angiogenesis is a phenomenon in animals in which vascular endothelial cells migrate and proliferate from existing blood capillaries and other vessels to form a new vascular network through lumen formation. During the wound healing process (inflammatory, proliferative, and maturation phases), new blood vessels are formed toward the injured area to transport oxygen and energy necessary for the healing of the injured area. Therefore, it is thought that wound healing can be accelerated by promoting angiogenesis.

Vascular endothelial growth factor (VEGF), for example, is known as to induce angiogenesis. However, VEGF is known to be produced by tumor cells, for example, and induces pathological angiogenesis (Patent Literature 1). There is also technology to generate endothelial cells from human induced pluripotent stem cells (iPS cells) (see Patent Literature 2). However, there is a possibility that transplantation of iPS cells with remaining undifferentiated cells may cause cancer, and there are safety issues with the use of this technology.

In addition, the transplantation of neural stem cells for the treatment of neurological diseases such as Parkinson's disease and spinal cord injury is also being considered due to a lack of donors and ethical issues (Non-Patent Literature 1, Patent Literature 3).

With the discovery of iPS cells, it is now possible, for example, to create iPS cells from the patients themselves and to induce neural stem cells from them. It is now expected that autologous transplantation, in which the patient's own cells can be used for treatment. However, it is believed that it takes more than half a year to create neural stem cells from the patient's own cells via iPS cells. In addition, the safety and direction of differentiation of iPS cell lines are considered to differ greatly from one cell line to another. It takes even longer to verify that an iPS cell is safe. Furthermore, in spinal cord injury, neural stem cells are considered to be ineffective unless they are transplanted before the patient's symptoms become fixed. Therefore, for example, in such diseases where cell transplantation is effective for a limited period, it would be difficult to use the patient's own cells for treatment even if iPS cell transplantation technology is used (Non-Patent Literature 1). In addition, even in the case of transplantation of such neural stem cells, the cell viability after such transplantation may be low (Non-Patent Literature 2).

### Citation List

### Non-Patent Literature

Non-Patent Literature 1: Takeshi Matsui et al. STEM CELLS 2012, 30: 1109-1119.
Non-Patent Literature 2: Koichi Iwatsuki: Spinal Surgery 20, 29: pp. 26-31.

### Patent Literatures

[Patent Literature 1] JP-A-2016-216375
[Patent Literature 2] WO2014/192925
[Patent Literature 3] JP-A-2005-278641

### Summary of Invention

### Technical Problem

There is still a need for safe means of promoting angiogenesis and/or nerve regeneration (e.g., techniques to induce angiogenesis in cells and tissues transplanted into the body), development of scaffolds and other agents to allow neural stem cells (undifferentiated tissue stem cells of the nervous system) to grow and proliferate after such transplantation, and the like.

Therefore, the object of this invention is to provide a safe means of promoting angiogenesis, and the like.

### Solution to Problem

The present invention was made to solve such problems, i.e., one of the inventions is "a unit for use in promoting angiogenesis and/or nerve regeneration, comprising a gel component and proteoglycans," and the invention includes, for example, the following (1) to (11) .
(1) A unit for use in promoting angiogenesis and/or nerve regeneration, the unit comprising a gel component and a proteoglycan.
(2) The unit according to (1), comprising VEGF.
(3) The unit according to (1) or (2), comprising FGF-2
(4) The unit according to any one of (1) to (3) , comprising EGF.
(5) The unit according to any one of (1) to (4), further comprising cells for promoting angiogenesis.
(6) The unit according to any one of (2) to (5), wherein a liquid medium containing VEGF and the cells for promoting angiogenesis are stacked on top of the gel component.
(7) The unit according to any one of (1) to (6), wherein a proteoglycan content is 0.1µg/ml or more.
(8) The unit according to any one of (2) to (7), wherein the proteoglycan is contained in a liquid medium including VEGF.
(9) The unit according to any one of (1) to (8), wherein the proteoglycan is contained in the gel component.
(10) The unit according to any one of (1) to (9), wherein the proteoglycan is a chondroitin sulfate-type proteoglycan.
(11) The unit according to (9), wherein the proteoglycan is a proteoglycan derived from salmon nasal cartilage.

### Effects of the Invention

By using this unit, for example, it is possible to safely promote angiogenesis in a human body by transplanting this unit into the body.

### Brief description of the drawings

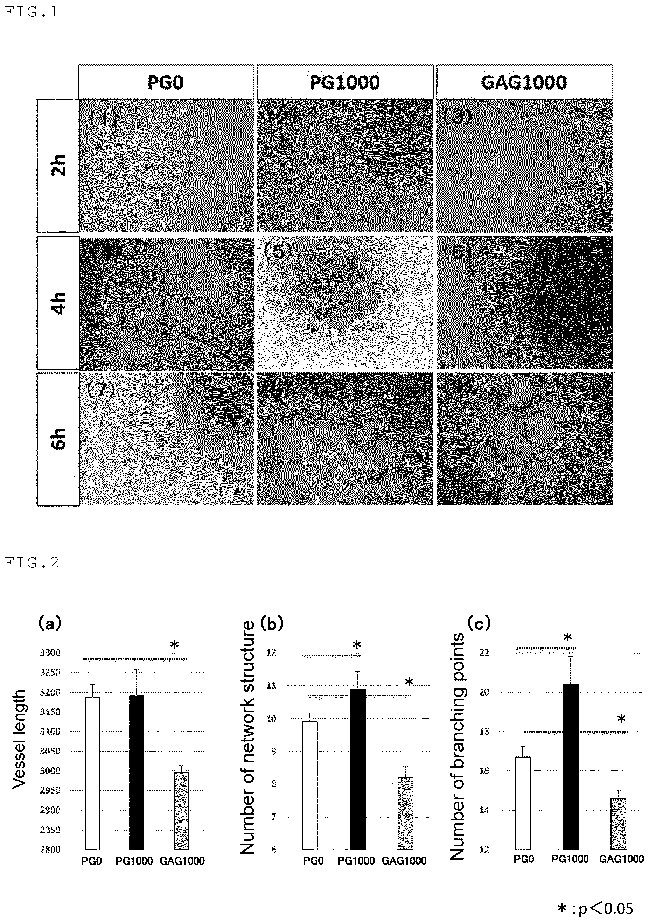

Fig.1 shows the results of evaluation of angiogenic potential (photographs taken by phase contrast microscopy at 4x magnification). In the figure, (1) is a photograph of the PG0 group at 2 hours after the start of culture, (2) is a photograph of the PG1000 group at 2 hours after the start of culture, (3) is a photograph of the GAG1000 group at 2 hours after the start of culture, (4) is a photograph of the PG0 group at 4 hours after the start of culture, (5) is a photograph of the PG1000 group at 4 hours after the start of culture, (6) is a photograph of the GAG1000 group at 4 hours after the start of culture, and (7) is a photograph of the PG0 group at 6 hours after the start of culture, (8) is a photograph of the PG1000 group at 6 hours after the start of culture, and (9) is a photograph of the GAG1000 group at 6 hours after the start of culture.
Fig.2 shows the results of the measurement of vessel length (a), number of network structures (b), and number of branching points (c) in the samples of each group in Fig.1 after 6 hours of culture. The results of these measurements were obtained by quantifying each item (length of blood vessels, number of network structures, and number of branching points) in four randomly selected fields of view, and then calculating the average of the quantified values. Statistical analysis was performed using the Dunnett's method, with ∗ indicating a significant difference, p<0.05. The measurement was performed by conducting triplicate cultures and calculating the average of the three times. The length of blood vessels (pixel) in each group was 3187 in the PG0 group, 3192 in the PG1000 group, and 2996 in the GAG1000 group (significantly different from the PG0 group). The number of network structures in each group was 9.9 in the PG0 group, 10.9 in the PG1000 group (significantly different from PG0 group), and 8.2 in the GAG1000 group (significantly different from PG0). The number of branching points in each group is 16.7 in the PG0 group, 20.4 in the PG1000 group (significantly different from the PG0 group), and 14.6 in the GAG1000 group (significantly different from the PG0 group).

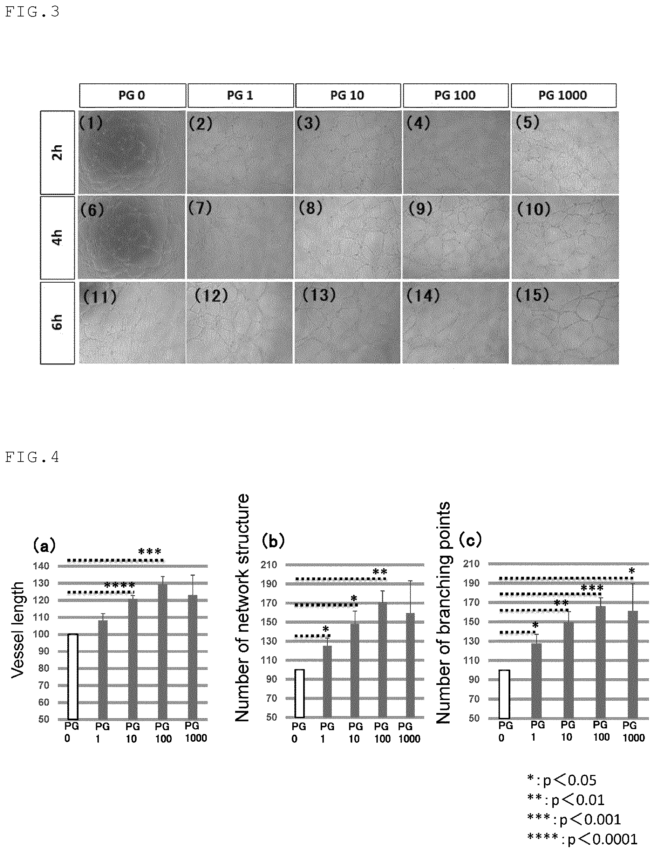

Fig.3 shows the results of evaluation of angiogenic potential (photographs taken by phase contrast microscopy at 4x magnification). In the figure, (1) is a photograph of PG0 group at 2 hours after the start of culture, (2) is a photograph of PG1 group at 2 hours after the start of culture, (3) is a photograph of PG10 group at 2 hours after the start of culture, (4) is a photo of PG100 group at 2 hours after the start of culture, (5) is a photograph of PG1000 group at 2 hours after the start of culture, (6) is a photograph of PG0 group at 4 hours after the start of culture, (7) is a photograph of PG1 group at 4 hours after the start of culture, (8) is a photograph of PG10 group at 2 hours after the start of culture, (9) is a photograph of the PG100 group at 4 hours after the start of culture, (10) is a photograph of PG1000 group at 4 hours after the start of culture, (11) is a photograph of the PG0 group at 6 hours after the start of culture, (12) is a photograph of the PG1 group at 6 hours after the start of culture, (13) is a photograph of PG10 group at 6 hours after the start of culture, (14) is a photograph of PG100 group at 6 hours after the start of culture, (15) is a photo of PG1000 group at 6 hours after the start of culture.
Fig.4 shows the results of measurements of the length of blood vessels (a), the number of network structures (b), and the number of branching points (c), in the samples after 6 hours of culture of each group shown in Fig.3. The measurements were made for three cultures by calculating the average of the three times. The results of this measurement were obtained by quantifying each item (length of blood vessels, number of network structures, and number of branching points) in four randomly selected fields of view, and calculating the average of the quantified values. In this measurement, the relative values are shown when PG0 is set to 100. In this measurement, statistical analysis was performed using the Dunnett's method, and ∗ indicates a significant difference, p<0.05 (∗ in the figure), p<0.01 (indicated by ∗∗ in the figure), p<0.001 (indicated as ∗∗∗ in the figure), p<0.0001 (indicated as ∗∗∗∗ in the figure). The length of the vessels in each group, with the PG0 group as 100, was 108.20 for the PG1 group, 120.87 for the PG10 group, (significantly different from the PG0 group), 129.44 for the PG100 group (significantly different from PG0 group), and 123.17 for the PG1000. The number of network structures in each group, with the PG0 group as 100, was 125.23 for the PG1 group (significantly different from the PG0 group), PG10 ), 148.17 for the PG10 group (significantly different from PG0), 170.8 for the PG100 group (significantly different from PG0), and 159.66 for the PG1000 group (significantly different from the PG0 group), the number of bifurcation points for each group is as follows: if the PG0 group is set to 100, the PG1 group was 127.68 (significantly different from PG0 group), PG10 group was 149.59 (significantly different from PG0), PG100 group was 166.2 (significantly different from PG0), and PG1000 group was 161.37 (significantly different from PG0).

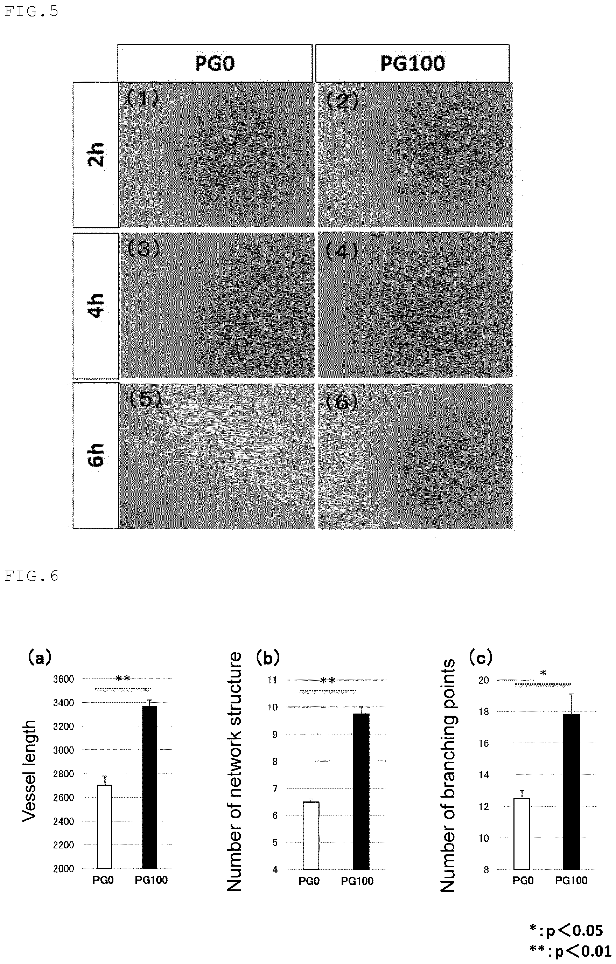

Fig.5 shows the results of evaluation of angiogenic potential (photographs taken by phase contrast microscopy at 4x magnification). In the figure, (1) is a photograph of the PG0 group at 2 hours after the start of culture, (2) is a photograph of the PG100 group at 2 hours after the start of culture, (3) is a photograph of the PG0 group at 4 hours after the start of culture, (4) is a photograph of the PG100 group at 4 hours after the start of culture, (5) is a photograph of the PG0 group at 6 hours after the start of culture, and (6) is a photograph of the PG100 group at 6 hours after the start of culture.
Fig.6 shows the results of measurements of the length of blood vessels (a), the number of network structures (b), and the number of branching points (c) in the samples at 6 hours after the start of culture for each of the groups shown in Fig.5. The results of this measurement were obtained by quantifying each item (length of blood vessels, number of network structures, and number of branching points) in four randomly selected fields of view and calculating the average of the quantified values. In this measurement, statistical analysis was performed using the Dunnett's method, and ∗ indicates a significant difference, p<0.05 (∗ in the figure), and p<0.01 (indicated by ∗∗ in the figure). The measurements were made for two cultures by calculating the average of the two measurements. The length of the vessels (pixel) in each group is 2702 in the PG0 group and 3368 in the PG100 group (significantly different from the PG0 group). The number of network structures in each group was 6.49 in the PG0 group and 9.75 in the PG100 group (significantly different from the PG0 group). The number of branching points in each group was 12.5 in the PG0 group and 17.8 in the PG100 group (significantly different from PG0).

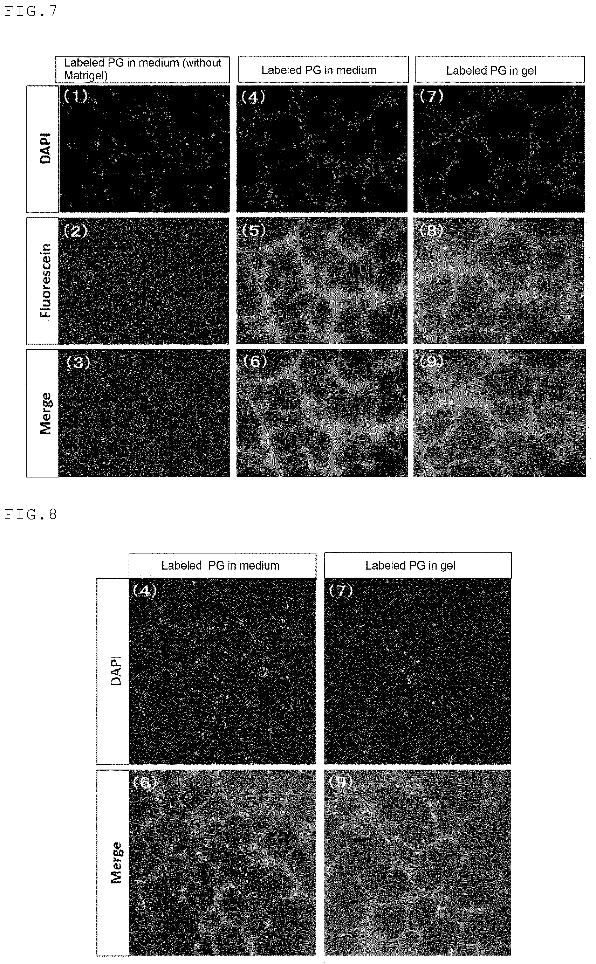

Fig.7 shows the results of Experiment 3 (confirmation of proteoglycan localization by fluorescence microscopy). In the figure, (1) is the result of DAPI staining of the labeled PG group in the medium (without Matrigel), (2) is the result of staining with labeled PG1 of the labeled PG group in the medium (without Matrigel), (3) is the result of Merge staining of labeled PG group in the medium (without Matrigel), (4) is the result of DAPI staining of the labeled PG group in the medium, (5) is the result of staining with labeled PG1 of the labeled PG group in the medium, (6) is the result of Merge staining of labeled PG group in the medium, (7) is the result of DAPI staining of the labeled PG group in the gel, (8) is the result of staining with labeled PG1 of the labeled PG group in the gel, and (9) is the result of Merge staining of labeled PG group in the gel. "DAPI" indicates that the nuclei of HUVECs were labeled by DAPI staining, and "Fluorescein" indicates that the proteoglycans were labeled with labeled PG1, and "Merge" indicates the combination of the labeling of the nuclei and the proteoglycans.
Fig.8 shows the results of Experiment 3 (confirmation of proteoglycan localization by confocal microscopy), an extract of (4), (6), (7), and (9) shown in Fig.7.

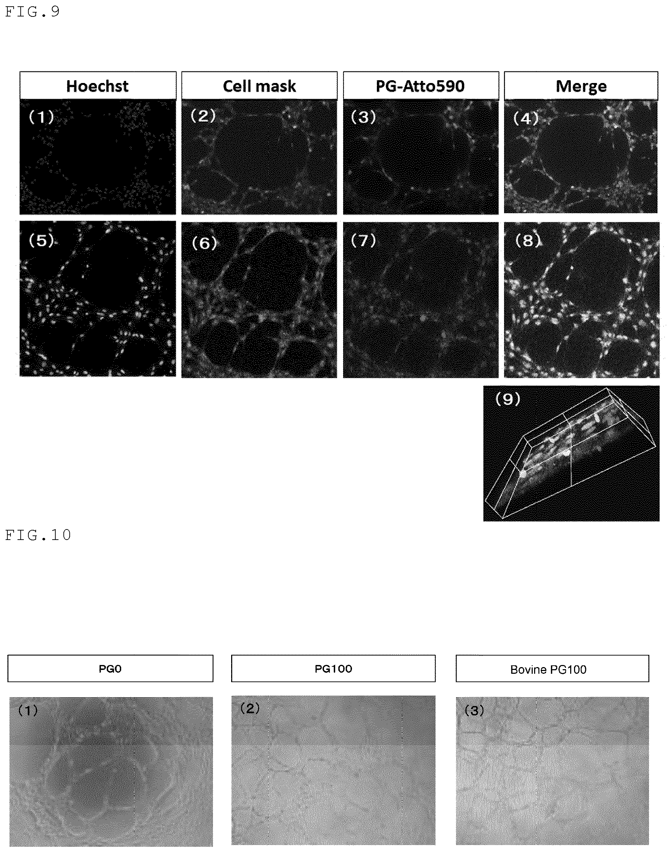

Fig.9 shows the results of Experiment 4, which confirmed the localization of proteoglycans by confocal microscopy. "Hoechst" indicates that the nuclei of HUVECs were labeled by Hoechst staining, "Cell mask" indicates that the cell membrane of HUVECs was labeled by CellMask^{™} staining, "PG-Atto590" indicates that the proteoglycans were labeled with labeled-PG2, and "Merge" indicates that the labeling of the nuclei, the cell membrane, and the proteoglycan were combined. (1) is the result of the microscopic observation of the "Hoechst" at 20x magnification, (2) is the result of the microscopic observation of the "Cell mask" at 20x magnification, (3) is the result of microscopic observation of "PG-Atto590" at 20x magnification, (4) is the results of the microscopic observation of "Merge" at 20x magnification, (5) is the results of microscopic observation of "Hoechst" at 40x magnification, (6) is the result of microscopic observation of "Cell mask" at 40x magnification, (7) is the result of microscopic observation of "PG-Atto590" at 40x magnification, (8) is the result of microscopic observation of "Merge" at 40x magnification, and (9) is the result of microscopic observation of "Merge" at 60x magnification.
Fig.10 shows the results of the evaluation of angiogenic potential in Experiment 5 (photographs taken by phase contrast microscopy at 4x magnification). In the figure, (1) is a photograph of the PG0 group at 6 hours after the start of culture, (2) is a photograph of the PG100 group at 6 hours after the start of culture, and (3) is a photograph of the bovine PG100 group at 6 hours after the start of culture.
Fig.11 shows the results of evaluating angiogenic potential using the aortic ring assay (results of Experiment 6).
Fig.12 shows the results of evaluating the regenerative potential of nerves and/or blood vessels using a sciatic nerve injury model (results of Experiment 7).
Fig.13 shows the results of evaluating angiogenic potential using the sciatic nerve injury model (results of Experiment 8).
Fig.14 shows the results of evaluation of angiogenic capacity and other parameters using the sciatic nerve injury model (results of Experiment 9). The bar shown in Fig.14 indicates 500pm. The dotted line shown in Fig.14 is the site of the injured sciatic nerve when the model mouse was created.
Fig.15 shows the results of evaluating angiogenic potential using the sciatic nerve injury model (results of Experiment 11).
Fig.16 shows the results of Experiment 12.
Fig.17 shows the result of Experiment 12. The bar shown in Fig.17 indicates 20µm.
Fig.18 shows the result of Experiment 12. The bar shown in Fig.18 indicates 200pm.
Fig.19 shows the schematic diagram of the experimental procedure performed in Experiment 13.
Fig.20 shows the measurement results of the number of neurospheres formed in each treatment group. The vertical axis indicates the percentage (%) of neurospheres (NS) formed, specifically, the number of NS formed in the control group was 100 (%), and the percentage of NS formation (%) is shown. Statistical analysis was performed using the Dunnett's method, and ∗ indicates a significant difference, p<0.05.
Fig.21 shows the observations of neurospheres in each treatment group.
Fig.22 shows a schematic diagram of the experimental procedure performed in Experiment 14.
Fig.23 shows a schematic diagram of the experimental procedure performed in Experiment 15.
Fig.24 shows a schematic diagram of the experimental procedure performed in Experiment 16.
Fig.25 shows results of Experiment 16 (confirmation of proteoglycan localization in neural stem cells). "The group of culture medium 2" is the group cultured under the condition of medium 2 (a group cultured in medium containing unlabeled proteoglycans), and "The group of culture medium 8" is the group cultured under the condition of medium 8 (a group cultured in medium containing labeled proteoglycans). "Hoechst" indicates that the nuclei of the neural stem cells were labeled by Hoechst staining. "Fluorescein-PG" indicates that localized proteoglycans were labeled by fluorescent staining.
Fig.26 shows the results of Experiment 17 (confirmation of proteoglycan localization in neural stem cells by confocal microscopy). "Hoechst" indicates that the nuclei of neural stem cells were labeled by Hoechst staining. "Cell mask" indicates that the cell membrane of the neural stem cell was labeled. "PG-Atto590" indicates that the proteoglycans were labeled, and "Merge" indicates that the labeling of the nuclei, the labeling of the cell membrane, and the labeling of the proteoglycans were combined.

### Description of Embodiments

The following is a description of the units involved in the invention.

### (VEGF)

Vascular endothelial growth factor (VEGF) is a growth factor that specifically affects vascular endothelial cells isolated from culture media of pituitary folliculo-stellate cell. VEGF is a member of the cysteine-knot growth factor superfamily and is a cell growth factor that plays an important role in de *novo* angiogenesis (vasculogenesis) during the embryonic period, and the formation of new blood vessels by branching and elongation of existing vessels (angiogenesis). VEGF is a member of the VEGF family of proteins, which includes VEGF-A, VEGF-B, and the like. VEGF-A has effects on vascular endothelial cell proliferation, enhancement of vascular permeability, promotion of ductal vaginogenesis, and induction of production of active substances from endothelial cells, while VEGF-E has specific effects on tumor tissue angiogenesis.

### (Liquid medium)

Liquid medium is preferred for the medium used in the present invention in terms of simplicity of medium preparation (e.g., it is considered easier to prepare the medium when performing floating culture). The liquid medium used can be any known medium. For example, a medium containing the components (inorganic salts, carbohydrates, hormones, essential amino acids, and vitamins) necessary for cell survival and growth (e.g. Iscove modified Dulbecco's medium (IMDM), RPMI, and DMEM (e.g., DMEM/Ham's F-12 medium (NACALAI TESQUE, INC. 08460-95) used in the following examples), Fischer's medium, α-medium, Leibovitz medium, L-15 medium, NCT L-15 medium, NCTC medium, F-12 medium, MEM, and McCoy medium). Further additives (serum, antibiotics, etc.) may also be included as needed.

### (Angiogenesis)

Angiogenesis is an important morphogenetic phenomenon that creates a network of new blood vessels *in vivo,* in which vascular endothelial cells collaborate with other cells to promote the formation of new blood vessels in a wound-specific manner. (in more detail, vascular endothelial cells cooperate with other cells to form characteristic vascular structures, such as two- and three-dimensional branching structures, through repeated budding, elongation, branching, and lumen formation.) *In vivo* phenomena that occur in normal (physiological) angiogenesis include fetal angiogenesis, endometriogenesis, follicle formation, and wound healing. *In vivo* phenomena that occur with pathological angiogenesis are, for example, malignant tumor formation, intraocular angiogenic disease, rheumatoid arthritis, and atherosclerosis. *In vivo* phenomena caused by inadequate angiogenesis include, for example, arteriosclerosis obliterans, angina pectoris, and myocardial infarction. To treat *in vivo* phenomena (diseases) caused by inadequate angiogenesis, vascular regeneration therapy is used, for example, to promote angiogenesis.

### (Cells to cause angiogenesis promotion)

Cells to cause angiogenesis promotion used in the present invention are, any kind of mammalian cells, such as human and other mammalian cells, for example, somatic cells, progenitor cells of such somatic cells, or mixed cells thereof. Cells for causing angiogenesis promotion used in the present invention include, for example, cardiomyocytes, endothelial cells (vascular endothelial cells, lymphatic endothelial cells, etc.), wall cells (pericytes, etc.), muscle cells (skeletal muscle cells, smooth muscle cells, etc.), etc., as somatic cells. In the following examples, HUVECs (human umbilical vein endothelial cells) were used as cells to promote angiogenesis.

### (Gel component)

The gel component (material) used in the present invention is usually biocompatible and can contain large amounts of water, allowing easy diffusion and transfer of substances necessary for cell survival, such as oxygen, water, nutrients and enzymes. The form of the gel component is not limited as long as it can be incorporated into the unit, for example, particulate, granular, film, tube, disk, mesh, mesh, porous, suspended or dispersed. Gel components include, for example, hydrogels such as gelatin hydrogels. The gel component can also contain various components, such as laminin, type IV collagen, entactin/nidogen, and predetermined growth factors, as needed to adjust the proliferative potential of cells to promote angiogenesis. In the examples below, the gel component is, for example, Matrigel (registered trademark, Corning, Product Number: 356231), and Collagen Gel Culturing Kit cellmatrix type1-A (Nitta Gelatin, 638-00781) was used.

### (Proteoglycan)

Proteoglycans are glycoproteins with sugar chains called glycosaminoglycans such as chondroitin sulfate and dermatan sulfate (hereinafter referred to as GAGs) covalently attached to the core protein. Proteoglycans are widely distributed in the body, including skin and cartilage, as one of the major components of the extracellular matrix. GAG chains have a long linear structure with no branches. Because of the large number of sulfate and carboxy groups, it is negatively charged, and the GAG chain takes on an extended shape due to its electrical repulsive force. In addition, proteoglycans can hold a large amount of water due to the water affinity of sugars. The large number of GAG chains in proteoglycans is responsible for the unique functions of cartilage, such as elasticity and resistance to impact, while flexibly holding water like a sponge.

The core protein of proteoglycans has the property of binding to various molecules in the matrix. In the case of cartilage proteoglycans, the N-terminus has a binding region for hyaluronan and link protein, which can bind to these substances, and also aggregate between the same molecules, and the C-terminus has lectin-like and EGF-like regions that can bind to various other molecules. These properties allow proteoglycans to develop structures that are unique to each tissue. Among proteoglycans, chondroitin sulfate-type proteoglycans are proteoglycans in which chondroitin sulfate is covalently bound to the core protein.

Proteoglycans derived from salmon nasal cartilage are proteoglycans extracted from salmon nasal cartilage. Here, salmon is, for example, a member of the genus Oncorhynchus, preferably with the scientific name "Oncorhynchus" in terms of cell proliferation (e.g., more efficient cultivation of the cells to be cultured). "Oncorhynchus keta" is selected for its cell proliferation (e.g., more efficient cultivation of the cells to be cultured).

The content of proteoglycans in the unit of the invention, for example, in terms of cell proliferative properties, has a lower limit of preferably 0.1µg/ml or more, more preferably 0.2ug/ml or more, and still more preferably 0.5ug/ml or more.

The proteoglycans contained in the unit of the present invention are prepared by the method described in, for example, the gazette (Japanese Patent No.6317053). In the experiments shown in the following examples, proteoglycans derived from salmon nasal cartilage (FUJIFILM Wako Pure Chemical Corporation (product codes: 162-22131, 168-22133)), proteoglycans derived from bovine nasal septum and the like were used.

In the units of the invention, proteoglycans are included in the liquid medium and/or gel component.

### (Unit)

As described above, the unit of the present invention is "unit (population) used for angiogenesis promotion, including a gel component and a proteoglycan", "unit (population) containing a liquid medium containing VEGF, cells to cause angiogenesis promotion, a gel component, and a proteoglycan" and the like. For example, it is also possible to create multiple stacked units by using further gel component, and transplant the stacked unit into human body or the like.

The unit of the present invention can be used, for example, in cell therapy such as cell transplantation (a therapy to treat diseases using one's own cells or cells of others), regenerative medicine (a therapy to induce functional tissue regeneration by local mobilization of stem cells existing *in vivo* to injured or diseased tissue without removing them from the body).

### (FGF-2)

FGF-2 (Fibroblast Growth Factor-2, also called bFGF) can be isolated from animal nervous tissue, pituitary gland, adrenal cortex, and placenta. FGF-2 is known to induce neural differentiation, survival, and regeneration, as well as regulate embryonic development and differentiation. FGF-2 has a wide range of functions, such as a cell growth factor, angiogenic factor, and neurotrophic factor, and shows proliferative activity against a variety of cells, including ES cells and iPS cells.

### (EGF)

EGF (Epidermal Growth Factor) is a polypeptide that promotes the proliferation of various cells including epithelial cells. EGF is not species specific and has proliferative effects on a variety of cells including epithelial cells, fibroblasts, and hepatocytes. *In vivo,* EGF is considered to be a growth factor that plays an important role in cell proliferation and
differentiation.

### (Neural stem cell)

Neural stem cells are undifferentiated tissue stem cells of the nervous system that combine self-renewal and pluripotency. In the embryonic brain of humans and other mammals, neural stem cells first proliferate actively, increasing their number exponentially, and then generating asymmetric division. It is also known to produce astrocytes and oligodendrocytes in the late fetal and postnatal brain. Neural stem cells are the source of neurons, astrocytes, and oligodendrocytes, the major cell types that make up the central nervous system. In the present invention, preferably, cells from mammals such as humans, mice, and the like are used.

### (Nerve regeneration)

Nerve regeneration, such as of central and/or peripheral nerves, represents at least partial reproduction of the process of normal development in nerves and is independent of the origin of the cells to be regenerated. Cells to be regenerated include, for example, stem cells (e.g., neural stem cells, embryonic stem cells, bone marrow cells, etc.), neural progenitor cells, or neurons. Furthermore, the nerve-regenerating cells may be intrinsic (e.g., neural stem cells, neural progenitor cells, neurons, mature neurons, etc.) or exogenous (e.g., transplanted neural stem cells, transplanted neural progenitor cells, transplanted neurons, transplanted mature neurons, etc.). Exogenous cells may be autologous cells or cells derived from other families. Neural regeneration encompasses tissue regeneration or functional regeneration, and includes, for example, cell viability, differentiation, proliferation and/or maturation as described above. Maturation is, for example, the growth of nerve cells to a functionally working state such as signal exchange. Regeneration also encompasses, for example, neurotrophic factor-like effects and enhanced neurotrophic factor activity. For example, peripheral nerve regeneration is the extension of peripheral nerves injured by external or internal factors to target cells, the ability to reconstruct nerve circuits, and the prevention and/or treatment of peripheral nerve disorders.

### Examples

### [Experiment 1: Evaluation of angiogenic potential]

Umbilical vein endothelial cells derived from human normal newborns (Human Umbilical Vein Endothelial Cells (HUVECs)) were used to evaluate the angiogenic potential of units containing proteoglycans and other substances.

The experimental procedure is described below. First, Matrigel (150µl/well) was placed in each well and the wells were incubated at room temperature (23°C) for 10 minutes. After this incubation, the wells were further incubated at 37°C and 5% CO₂ for 30 minutes . After this 30-minute incubation, culture medium (EGM^{™}-2 BulletKit^{™}) was added to each well and equilibration was performed at 37°C, 5% CO₂ for 15 minutes. After the equilibration, HUVECs (5.0 × 10⁴ cells/well) were inoculated to each well for the following groups (PGO, PG1, PG10, PG100, PG1000, and GAG1000) under the conditions of the culture medium at 37°C, 5% CO₂. Two hours after the start of this culture (indicated as 2h in Fig.1), four hours after the start of this culture (indicated as 4h in Fig.1) , and after 6 hours (indicated as 6h in Fig.1), the specified evaluations were performed.

In this Experiment 1, the composition of the medium of each group (PGO, PG1, PG10, PG100, PG1000, and GAG1000) is as follows.

### (PG0)

No proteoglycans in culture medium (EGM^{™}-2 BulletKit^{™}) .

### (PG1)

Culture medium (EGM^{™}-2 BulletKit^{™}) to which the proteoglycan was added at a final concentration of 1µg/ml.

### (PG10)

Culture medium (EGM^{™}-2 BulletKit^{™}) to which the proteoglycan was added at a final concentration of 10µg/ml.

### (PG100)

Culture medium (EGM^{™}-2 BulletKit^{™}) to which the proteoglycan was added at a final concentration of 100µg/ml.

### (PG1000)

Culture medium (EGM^{™}-2 BulletKit^{™}) to which the proteoglycan was added at a final concentration of 1000ug/ml.

### (GAG1000)

Culture medium (EGM^{™}-2 BulletKit^{™}) to which GAG was added at a final concentration of 1000ug/ml.

The results of this Experiment 1 are shown in Figs.1 to 4. The experimental results shown in Figs.1 and 2 indicate that the addition of proteoglycans to the medium promotes angiogenesis, but the addition of GAGs (which, unlike proteoglycans, the core protein is not bound thereto) did not promote angiogenesis. The experimental results shown in Figs.3 and 4 indicate that the concentration-dependent addition of proteoglycans to the medium (at least up to the group with 100µg/ml of proteoglycans) resulted in enhanced angiogenesis.

### [Experiment 2: Evaluation of angiogenic potential]

HUVEC was used to evaluate the angiogenic potential of the unit containing the proteoglycan and other substances. Unlike Experiment 1, in this Experiment 2, the angiogenic potential was evaluated by adding a predetermined amount of the proteoglycan to Matrigel.

The experimental procedure is described below. In each well, Matrigel (150pl/well, without proteoglycan) or Matrigel with the proteoglycan added at 100ug/ml (150µl/well) were allowed to stand at room temperature (23°C) for 10 minutes. Here, in Figs. 5 and 6, "PG0" refers to the group of Matrigel (150µl/well, without proteoglycan), "PG100" refers to the group of Matrigel with the proteoglycan added at 100ug/ml (150µl/well). After this incubation, the culture was further incubated at 37°C and 5% CO₂ for 30 minutes. After this 30-minute incubation, culture medium (EGM^{™}-2 BulletKit^{™}) was added to each well and equilibrated at 37°C, 5% CO₂ for 15 minutes. After the equilibration, HUVECs (5.0 × 10⁴ cells/well) were inoculated in each well and incubated at 37°C, 5% CO₂ . The evaluation was performed at 2 hours (indicated as 2h in the figure), 4 hours (indicated as 4h in the figure), and 6 hours (indicated as 6h in the figure) after the start of the culture.

The results of this Experiment 2 are shown in Figs.5 and 6. The experimental results shown in Figs.5 and 6 indicate that angiogenesis is promoted even when proteoglycans are added to Matrigel rather than culture medium.

### [Experiment 3: Confirmation of proteoglycan localization in angiogenesis]

HUVECs were used to confirm the localization of proteoglycans in angiogenesis using fluorescence and confocal microscopy.

The procedure for this Experiment 3 is described below. First, prepare the following three types of wells.
- The group without Matrigel: In Fig. 7, this group is indicated as "Labeled PG in medium (without Matrigel)".
- The group to which Matrigel was added (70µl/well): In Figs.7 and 8, this group is indicated as "Labeled PG in medium".
- The group to which Matrigel was added (70µl/well). In addition, to this Matrigel, "Labeled PG1" was added to reach a final concentration of 100ug/ml in the unit of Matrigel and the culture medium: In Figs.7 and 8, this group is indicated as "Labeled PG in gel".

After these wells were prepared, they were allowed to stand at room temperature (23°C) for 10 minutes. After this incubation, the wells were further incubated at 37°C and 5% CO₂ for 30 minutes.

After this 30-minute incubation, the culture medium (EGM^{™}-2 BulletKit^{™}) was added to each well and equilibrated for 15 minutes at 37°C and 5% CO₂. After the equilibration, each well was inoculated with HUVECs (5.0 × 10⁴ cells/well). Here, in the group to which Matrigel was added (70µl/well) (The group of Labeled PG in medium), "Labeled PG1" was further added to the culture medium to reach a final concentration of 100µg/ml in the unit of Matrigel and culture medium.

After the inoculation, cells were cultured for 2 hours at 37°C, 5% CO₂. After the culture, cells in each well were collected by centrifugation at 400 × g for 3 minutes. The collected cells were washed with PBS for 5 minutes. After the wash, the cells were fixed in 4% paraformaldehyde for 15 minutes. After the fixation, cells were again washed with PBS for 5 minutes. After the wash, the cells were mounted on glass slides with DAPI-containing encapsulating agent (Dojin Kagaku Kenkyusho Co., Ltd. D212-Cellstain^{™}-DAPI) to prepare observation samples. Fluorescence microscopy (Olympus Corporation: Inverted Research Microscope IX81) and confocal microscopy (Olympus Corporation: Confocal Laser Scanning Microscope FV3000) were used at 40x magnification, to compare HUVEC nuclei (stained with DAPI), and the localization of proteoglycans.

Observations are shown in Figs.7 and 8. In Figs.7 and 8, blue indicates that the nuclei of HUVECs were stained with DAPI, and green indicates that proteoglycans were labeled. In the group "Labeled PG in medium (without Matrigel)", the localization of proteoglycans could not be found (Fig.7, (1) to (3)). On the other hand, in the groups "Labeled PG in medium" and "Labeled PG in gel" (Fig.7 (4) to (9) and Fig.8), it was found that the localization of endothelial cells and that of proteoglycans showed a high correlation (specifically, proteoglycans were distributed along the vessels that were trying to form a ring structure) already 2 hours after the start of the cell culture.

### [Experiment 4: Confirmation of proteoglycan localization in angiogenesis]

Using HUVECs, the localization of proteoglycans in angiogenesis was confirmed using fluorescence and confocal microscopy. The labeled proteoglycan used in this Experiment 4 was not the labeled PG2 used in Experiment 3, but the labeled PG1.

The procedure for this Experiment 4 is described below. First, prepare the following two types of wells.
- The group without Matrigel: this group is not shown in Fig.9.
- The group to which Matrigel was added (70pl/well), and in addition, proteoglycan was added to this Matrigel to a final concentration of 100µg/ml in the unit of Matrigel and culture medium: this group is illustrated in Fig.9.

After these wells were prepared, they were allowed to stand at room temperature (23°C) for 10 minutes. After this incubation, the wells were further incubated at 37°C, 5% CO₂ for 30 minutes.

The culture medium (EGM^{™}-2 BulletKit^{™}) was added to each well and equilibrated for 15 minutes at 37°C and 5% CO₂. After the equilibration, each well was inoculated with HUVECs (5.0 × 10⁴ cells/well). Here, in the group to which Matrigel was added (70µl/well) (The group of Labeled PG in medium), "Labeled PG1" was further added to the culture medium to reach a final concentration of 100µg/ml in the unit of Matrigel and culture medium.

After the inoculation, cells were cultured for 2 hours at 37°C, 5% CO₂. One hour before collecting the cells to be used for observation (after the culture), the nuclei of the cells were stained with Hoechst 33342 (final concentration 10mg/mL. Hoechst^{™} 33342 Imaging protocol (Thermo Fisher Scientific) and Cellmask (final concentration 5mg/mL, the CellMask^{™} staining (Thermo Fisher Scientific) was added and stained. After the incubation, cells were collected by centrifugation at 400g for 3 minutes. The collected cells were washed with PBS for 5 minutes. After the wash, the cells were fixed in 4% paraformaldehyde for 15 minutes. After the fixation, the cells were washed again with PBS for 5 minutes. After the wash, the cells were mounted on glass slides with an encapsulant to prepare observation specimens. A confocal microscope (Olympus Corporation: Confocal Laser Scanning Microscope FV3000) was used to compare the HUVEC nuclei (stained with Hoechst as described above), the HUVEC cell membrane (stained with Cellmask), and proteoglycan localization, with magnifications of 20x, 40x and 60x.

The observations are shown in Fig.9. In Fig.9, "Hoechst" indicates that the nuclei of HUVECs were labeled by Hoechst staining, and "Cell mask" indicates that cell membranes of HUVECs were labeled by CellMask^{™} staining, and "PG-Atto590" indicates that proteoglycans were labeled with labeled PG2, and "Merge" indicates that the labeling of the nuclei, the labeling of the cell membranes, and the labeling of the proteoglycans were combined. Fig.9 confirms that, similar to the results shown in Figs.7 and 8, we confirmed that the localization of endothelial cells and proteoglycans showed a high correlation (specifically, proteoglycans were distributed along the blood vessels that were trying to form a ring structure) already two hours after the start of the cell culture. Although not shown in Fig.9, the localization shown in Fig.9 could not be confirmed in the group to which Matrigel was not added.

### [Experiment 5: Evaluation of angiogenic potential]

HUVECs were used to evaluate the angiogenic potential of the unit containing proteoglycans and other substances.

The experimental procedure is described below. First, Matrigel (150µl/well) was placed in each well and the wells were allowed to stand at room temperature (23°C) for 10 minutes. After this incubation, the wells were further incubated at 37°C, 5% CO₂ for 30 minutes. After this 30-minute incubation, culture medium (EGM^{™}-2 BulletKit^{™}) was added to each well and equilibrated at 37°C, 5% CO₂ for 15 minutes. After the equilibration, HUVECs (5.0 × 10⁴ cells/well) were inoculated in each well for each of the following groups (PGO, PG100, bovine PG100), the cells were cultured under the conditions of 37°C, 5% CO₂ medium. At 6 hours after the start of this culture, the specified evaluations were performed.

In this Experiment 5, the medium composition of each group (PGO, PG100 and bovine PG100) is as follows.

### (PG0)

Culture medium (EGM^{™}-2 BulletKit^{™}), without proteoglycan.

### (PG100)

Culture medium (EGM^{™}-2 BulletKit^{™}) to which proteoglycan (proteoglycan derived from salmon nasal cartilage) are added at a final concentration of 100ug/ml.

### (Bovine PG100)

Culture medium (EGM^{™}-2 BulletKit^{™}) to which proteoglycans derived from bovine nasal septum are added at a final concentration of 100ug/ml.

The results of this Experiment 5 are shown in Fig.10 and in Tables 1 to 3 below. In Tables 1 to 3, we show the results of the measurements of vessel length (Table 1), number of network structures (Table 2), and number of branching points (Table 3) in the samples from each group shown in Fig.10. The results shown in Tables 1 to 3 are the average of the quantified values for each of the four randomly selected fields of view (length of blood vessels, the number of network structures, and the number of branching points). In Tables 1 to 3, the measured values are relative values with PG0 as 100, and SD is the standard deviation.

The results of Experiment 5 showed that not only the addition of proteoglycans derived from salmon nasal cartilage to the medium (PG100), but also the addition of bovine nasal septum-derived (bovine PG100), promoted angiogenesis.

**[Table 1]**

| Vessel length | | |
|---|---|---|
| Experimental group | Measurements | SD |
| PG0 | 100 | 0 |
| PG100 | 136.7 | 4.6 |
| Bovine PG100 | 135.5 | 2.3 |

**[Table 2]**

| Number of network structures | | |
|---|---|---|
| Experimental group | Measurements | SD |
| PG0 | 100 | 0 |
| PG100 | 175.9 | 6.5 |
| Bovine PG100 | 176.3 | 6.6 |

**[Table 3]**

| Number of branching points | | |
|---|---|---|
| Experimental group | Measurements | SD |
| PG0 | 100 | 0 |
| PG100 | 170.2 | 12.3 |
| Bovine PG100 | 173.3 | 9.2 |

The following samples and other materials were used in the Experiment 1 to Experiment 5.
- HUVEC (C2517A and C2517AS, LONZA): Umbilical vein endothelial cells derived from human normal newborn (Human Umbilical Vein Endothelial Cells), cells derived from a single donor.
- EGM^{™}-2 BulletKit^{™} (CC-3162, LONZA) : When the basic medium EBM^{™}-2 (CC-3162, LONZA) is 500mL, VEGF (0.5mL), hEGF (0.5mL, human-derived EGF), R3-IGF-1 (0.5mL), ascorbic Acid (0.5mL), hydrocortisone (0.2mL), hFGFβ (2mL, EGF beta from human), heparin (0.5mL), FBS (10mL), and GA (0.5mL) are contained.
- Proteoglycan (FUJIFILM Wako Pure Chemical Corporation (Product Code: 162-22131, 168-22133): Proteoglycan, derived from salmon nasal cartilage. Only GAG was purified from this proteoglycan by the usual method and used in Experiment 1. Unlike proteoglycans, GAGs do not have a core protein bound to them.
- Fluorescently labeled proteoglycan 1 (also described in the specification and drawings as "labeled PG1"): ATTO 488 NHS ester (Sigma-Aldrich) and the proteoglycans were combined by the method recommended by the manufacturer(shown in the product: ATTO 488 NHS ester (Sigma-Aldrich)), and proteoglycans were reacted at room temperature for 2 hours. After this reaction, Zeba Spin Desalting Column (Thermo Fisher Scientific) was used for purification and fluorescently labeled proteoglycan was recovered. "Labeled PG1" is the recovered fluorescently labeled proteoglycan.
- Fluorescently labeled proteoglycan 2 (also described in the specification and drawings as "labeled PG2"): ATTO 590 (Sigma-Aldrich 70425) and the proteoglycans were combined by the method recommended by the manufacturer(shown in the product: ATTO 590 (Sigma-Aldrich 70425)), and proteoglycans were reacted at room temperature for 2 hours. After this reaction, Zeba Spin Desalting Column (Thermo Fisher Scientific) was used for purification and fluorescently labeled proteoglycan was recovered. "Labeled PG2" is the recovered fluorescently labeled proteoglycan.
- Matrigel (Corning, Product Number: 356231): Corning^{™} Matrigel Basement Membrane Matrix Growth Factor Reduced Phenol red free.
- Proteoglycan from the bovine nasal septum (Sigma-Aldrich, product number: P5864): proteoglycan from bovine nasal septum (Proteoglycan from bovine nasal septum chromatographically purified)

### [Experiment 6: Evaluation of angiogenic potential using the aortic ring assay]

To confirm the effects of the unit containing VEGF, gel components (collagen gel), and proteoglycans, thoracic aorta of 12-week-old male vascular reporter mice (Flt1-tdsRed BAC transgenic mice) were used to perform an aortic ring assay (*ex vivo* assay). The assay was published in an article (THE JOURNAL of JAPANESE COLLEGE of ANGIOLOGY Vol.45 No.10, pp. 637 to 641).

First, a layer of collagen gel (Base Layer) was prepared on a glass bottom dish.

Next, a ring-shaped aortic fragment (approximately 2mm thick) was prepared for use in the experiment. The thoracic aorta was collected from the reporter mouse, and the outer membrane was removed from the collected thoracic aorta to prepare the aortic fragment.

Next, approximately two aortic fragments were placed on the dish perpendicular to the bottom of the culture dish, and the reconstituted collagen solution (0.3 mass% Cellmatrix type1-A, Nitta Gelatin) was layered on the dish. After the layering, the dish was left at 37°C for 30 minutes, and the mixture (Base Layer, aorta fragments and reconstituted collagen solution) on the dish was gelatinized by leaving it at 37°C for 30 minutes after the layering.

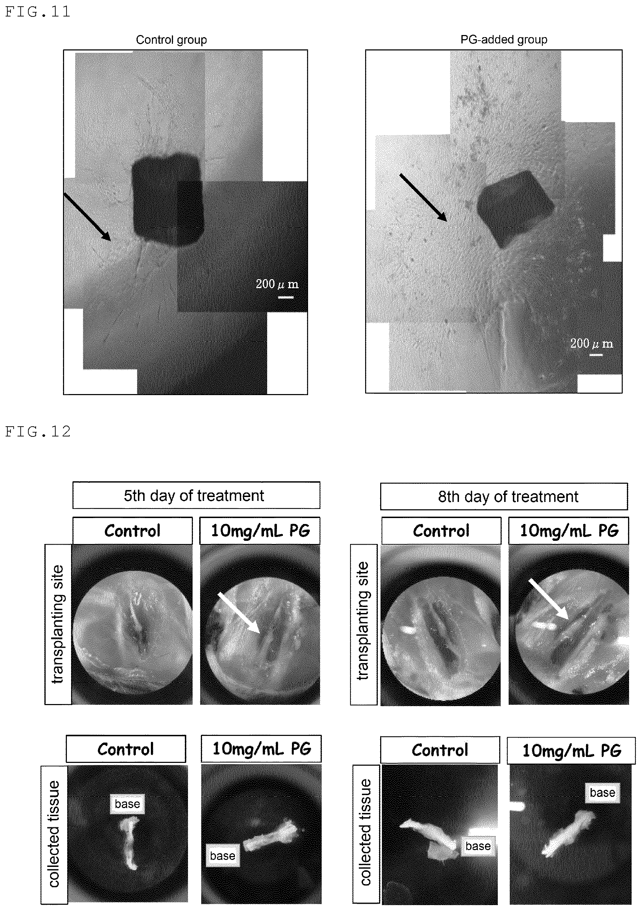

After the gelatinization, the control culture medium or the culture medium containing proteoglycans was added to the mixture. After the addition, the mixture to which the culture medium was added was incubated at 37°C for 14 days. The group to which the control culture medium was added was designated as "control group" and the group to which the culture medium containing proteoglycans was added was designated as "PG-added group". Cells elongated from the aortic fragments were checked by phase contrast microscopy. The total area of cells (indicated by arrows in Fig.11) extended from the aorta fragment was measured for the control group and the PG-added group. The bar in Fig.11 indicates 200um. The value of the total area was the result of the measurement of angiogenic potential.

The results of the measurements are described below. When the total area of the control group was set at 100, the PG-added group was 555.2 (Student's t-test, p<0.001). Thus, the PG-added group showed significantly greater angiogenic activity than the control group.

The following samples were used in this experiment 6.
- Collagen Gel: Collagen Gel Culturing Kit cellmatrix type1-A (Nitta Gelatin, 638-00781)
- Glass bottom dish: 35mm glass bottom DISH (IWAKI, 3911-035)
- Control culture medium (control group culture medium): In the case of 500mL basic culture medium, the following is added to the basic culture medium: Six types of solutions included in HuMedia-EG growth additive set, with a final concentration of 20ng/mL VEGF (Peprotech, Inc. 3624436), but without proteoglycans.
- Proteoglycan-containing culture medium (culture medium for PG-added group): In the case of 500mL of basic medium, six solutions included in the HuMedia-EG growth additive set, VEGF (Peprotech, Inc. 3624436) at a final concentration of 20ng/mL and proteoglycans at a final concentration of 100ug/mL.
- Basic medium: HuMedia-EB2 (Kurashiki Spinning Co., Ltd., KE-2350S)
- HuMedia-EG growth additive set (Kurashiki Boseki Co., Ltd., KE-6150): A kit of growth additives for normal human vascular endothelial cells (for 500ml). The kit contains FBS (10mL), hEGF (0.5mL, human-derived EGF), Hydrocortisone (hydrocortisone, 0.5mL), hFGFβ (0.5mL, human-derived EGF beta), Heparin (0.5mL), and an antibacterial agent (0.5mL, gentamicin/amphotericin B).
- Proteoglycan (FUJIFILM Wako Pure Chemical Corporation (Product Code: 162-22131, 168-22133)): Proteoglycan, derived from salmon nasal cartilage.

### [Experiment 7: Evaluation of nerve and/or blood vessel regenerative capacity using a sciatic nerve injury model]

Using a model mouse with an injured sciatic nerve (sciatic nerve transected), we evaluated the ability of the unit containing VEGF, gel components (Matrigel) and proteoglycans to regenerate nerves and/or vascular regeneration capacity.

Experimental methods are described. First, 12-week-old male vascular reporter mice (Flt1-tdsRed BAG transgenic mouse) were created by transecting the sciatic nerves (sciatic nerves of the right and left limbs) toward the tail of the mouse (n=2). The right sciatic nerve of the model mice was treated with 10mg/mL PG group, and the left sciatic nerve of the model mouse was treated with Control group.

Here, the following definitions are used.
- 10mg/mL PG group treatment: A unit containing proteoglycan (final concentration of 10mg/mL) was placed on the sciatic nerve (at the site of injury (amputation) of the sciatic nerve).
- Control group treatment: Matrigel was placed on the sciatic nerve (site of injury (transection) of the sciatic nerve).
- Proteoglycan (FUJIFILM Wako Pure Chemical Corporation (Product Code: 162-22131, 168-22133)): Proteoglycan derived from salmon nasal cartilage.
- Matrigel (Corning, Product Number: 356231): Corning^{™} Matrigel Basement Membrane Matrix Growth Factor Reduced Phenol-Red free.

Immediately after the treatment, the muscle layer and skin of the site of the treated model mice were sutured. After the suture, the model mice were reared normally for 5 days or 8 days. After the normal rearing, the model mice were dissected, and the treated areas were checked by the naked eye and stereomicroscope. After such confirmation, the sciatic nerve (about 5mm) was collected from the model mice.

FIG. 12 shows the result of the confirmation and photographs of the collected sciatic nerves.

In this confirmation, compared to the treatment of control group (labeled as Control in FIG.12, group containing no proteoglycan), the treatment of 10mg/mL PG group (labeled as 10mg/mL PG in FIG.12) showed regeneration of the sciatic nerve after 5 days of treatment (arrowed area in Fig.12). On the 8th day after treatment, more sciatic nerve regeneration was confirmed in the 10mg/mL PG group treatment than in the control group treatment (group containing no proteoglycan). No inflammation was observed in the vicinity of the regenerated site with the naked eye. When the collected sciatic nerves were confirmed, the sciatic nerves of the PG group treated with 10mg/mL were larger than the sciatic nerves of the control group treatment (group containing no proteoglycan) on both the 5th day and 8th day of treatment.

### [Experiment 8: Evaluation of angiogenic potential in a sciatic nerve injury model]

Using sciatic nerve injury model mice (sciatic nerve transected model mice), *in vivo* imaging was used to evaluate the ability to regenerate blood vessels by administering the unit containing a gel component (Matrigel) and proteoglycan. Evaluation using the imaging was performed using the IVIS Imaging System (PerkinElmer) including equipment.

Experimental methods are described. First, 9-week-old female vascular reporter mice (Flt1-tdsRed BAG transgenic mouse) were prepared by transecting the sciatic nerves (sciatic nerves of the right and left limbs) toward the tail of the mouse (n=1). The right sciatic nerve of the model mice was treated with 10mg/mL PG group, and the left sciatic nerve of the model mice was treated with the Control group.

Here, the following definitions are used.
- PG group treatment: Treatment in which the unit containing a proteoglycan (final concentration of 10mg/mL) in Matrigel is placed on the sciatic nerve. (the site where the sciatic nerve was injured (severed)).
- Control group treatment: Treatment in which Matrigel is placed on the sciatic nerve (the site where the sciatic nerve was injured (severed)).
- Proteoglycan (FUJIFILM Wako Pure Chemical Corporation (Product Code: 162-22131, 168-22133)): Proteoglycan, derived from salmon nasal cartilage.
- Matrigel (Corning, Product Number: 356231): Corning^{™} Matrigel Basement Membrane Matrix Growth Factor Reduced Phenol red free.

Immediately after the treatment, the muscle layer and skin of the site of the treated model mice were sutured. After said suture, the alfalfa-free fluorescence imaging feed (5V5M, PicoLab^{R} SelectMouse 50IF/9F) was also used for 7 days of normal rearing of the model mice.

On the seventh day of the breeding, the AngioSense^{™} 750EX (PerkinElmer, NeV10011EX) was injected into the tail vein of the model mice (dose of 2nmol/100pL of AngioSense^{™} 750 EX per mouse). Normal rearing was continued for a certain period of time after the injection. The IVIS of the model mice were photographed at 18, 21, and 24 hours after the injection.

The conditions for such photography are shown below.
- Exposure: 5.00 sec.
- Bining: Medium
- Fstop: 2
- Excitation: 640
- Emission: Cy5.5

Immediately before the imaging, the model mice were given general anesthesia, and after the anesthesia, body hair was removed from the predetermined area of the model mice. After the hairs were removed, the images were taken. The general anesthesia was performed by placing the model mice in a suction anesthesia box with anesthesia circulating for a certain period of time.
The removal was performed in the vicinity of the site where the above-mentioned treatment (PG group treatment or Control group treatment).

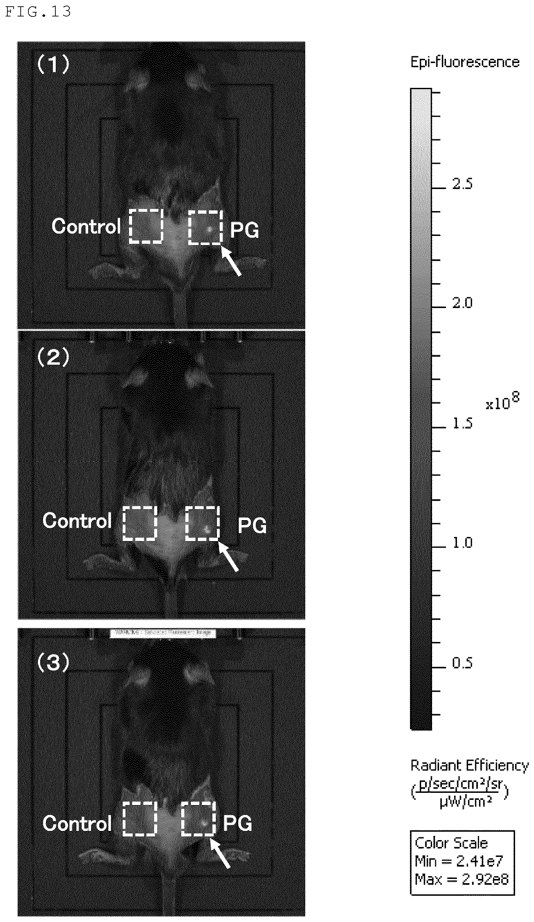

The results taken are shown in Fig.13. In Fig.13, (1) is the result after 18 hours, (2) after 21 hours, and (3) after 24 hours. The right side of Fig.13 shows the degree of fluorescence (Epi-fluorescence shown in Fig.13). The degree of fluorescence is calculated as a function of the radiant efficiency (equation in Fig.13, "radiant efficiency"). The yellow color indicates that more angiogenesis is taking place in the mouse. The yellow color in the group of PG group treatment (labeled PG in Fig.13) was significantly more pronounced than in the group of control group treatment (labeled Control in Fig.13). This expression indicates that angiogenesis is occurring.

In the evaluation shown in Fig.13, Total Radiant Efficiency ([p/s]/[µW/cm²], luminous intensity of the light source) and Average Radiant Efficiency ([p/s/cm²/sr]/[µW/cm²], the average luminance on the skin surface emitted from the light source) were also quantified in the area indicated by the square in Fig.13. The results are shown in Tables 4 and 5. In Tables 4 and 5, the symbols "(1), (2), (3), Control, PG" refer to the groups marked in Fig.13. The results of the quantitative analysis showed that the yellow color was significantly more pronounced in the PG treatment group than in the Control group.

**[Table 4]**

| Total Radiant Efficiency ([p/s]/[µW/cm²]) | | |
|---|---|---|
| | Control | PG |
| (1) | 2.27 x 10⁸ | 3.08 x 10⁸ |
| (2) | 2.56 x 10⁸ | 3.13 x 10⁸ |
| (3) | 2.36 x 10⁸ | 3.63 x 10⁸ |

**[Table 5]**

| Average Radiant Efficiency ([p/s/cm²/sr]/[µW/cm²]) | | |
|---|---|---|
| | Control | PG |
| (1) | 5.27 x 10⁷ | 7.14 x 10⁷ |
| (2) | 5.93 x 10⁷ | 7.25 x 10⁷ |
| (3) | 5.46 x 10⁷ | 8.42 x 10⁷ |

### [Experiment 9: Evaluation of angiogenic capacity, etc., using a sciatic nerve injury model]

Using a model mouse with an injured sciatic nerve (sciatic nerve transected), we checked whether administration of the unit containing a gel component (Matrigel) and proteoglycan causes angiogenesis and nerve regeneration by immunohistochemical staining.

Experimental methods are described. First, 12-week-old female vascular reporter mice (Flk1-GFP-BAC transgenic mice) were prepared by transecting the sciatic nerves (sciatic nerves of the right and left limbs) toward the tail of the mouse (n=1). The right sciatic nerve of the model mice was treated with 10mg/mL PG group, and the left sciatic nerve of the model mice was treated with the Control group.

Here, the following definitions are used.
- PG group treatment: Treatment in which the unit containing a proteoglycan (final concentration of 10mg/mL ) in Matrigel is placed on the sciatic nerve. (the site where the sciatic nerve was injured (severed)).
- Control group treatment: Treatment in which Matrigel is placed on the sciatic nerve (the site where the sciatic nerve was injured (severed)).
- Proteoglycan (FUJIFILM Wako Pure Chemical Corporation (Product Code: 162-22131, 168-22133)): Proteoglycan, derived from salmon nasal cartilage.
- Matrigel (Corning, Product Number: 356231): Corning^{™} Matrigel Basement Membrane Matrix Growth Factor Reduced Phenol red free.

Immediately after the treatment, the muscle layer and skin of the site of the treated model mice were sutured. After said suture, the alfalfa-free fluorescence imaging feed (5V5M, PicoLab^{R} SelectMouse 50IF/9F) was also used for 8 days of normal rearing of the model mice. After 8 days of normal rearing, the sciatic nerves of the right and left limbs of the model mice were collected. The right limb was the limb treated with the 10mg/mL PG group, and the left limb was the limb treated with the Control group.

The sciatic nerve of the right limb and the sciatic nerve of the left limb that were collected were washed in PBS for 5 min. After the wash, the cells were fixed in 4% paraformaldehyde for 1 hour. After the fixation, the cells were replaced with a 30 mass% sucrose solution. After the replacement, the sciatic nerve of the right limb and the sciatic nerve of the left limb were embedded using an embedding agent for frozen tissue section preparation (Tissue Tech O.C.T. Compound, Sakura Finetech Japan). Samples were prepared. After the embedding, the embedded samples were placed at -80°C for 3 hours. The frozen sections of the sciatic nerve of the right limb and the sciatic nerve of the left limb were prepared by this incubation. Cryostat HM525NX (Thermo Fisher Scientific) was used to cut the frozen sections to prepare sections at 100pm thickness.

The 100um-thick sections were blocked with blocking buffer for 3 hours at room temperature (about 25°C). After blocking, the sections were incubated with a reaction solution (primary antibody reaction solution) prepared by diluting a predetermined antibody with 10% Donkey Serum/PBS, and the primary antibody reaction was performed overnight (about 8 hours) at 4°C. After the primary antibody reaction, the antibody was intercepted using 0.3% TritonX-100/PBS (5 min at room temperature x3 times). After the washing, the sections were diluted with 10% Donkey Serum/PBS (secondary antibody reaction solution) for 3 hours at room temperature. The secondary antibody reaction was performed for 3 hours at room temperature. After the secondary antibody reaction, the antibody was intercepted using 0.2% TritonX-100/PBS (5 min at room temperature x1 time). After washing, the sections were sealed using the specified mounting agent. The sections after sealing were examined using a confocal microscope (Olympus Corporation: Confocal Laser Scanning Microscope FV3000) at a magnification of 4x to check whether the angiogenesis and nerve regeneration occurred.

The composition of the liquid used in the preparation of such sections described above is as follows.

**[Table 6]**

| Blocking buffer (composition of 5mL) | |
|---|---|
| Component | Content |
| 10 mass% of Triton X-100 | 150µL |
| Donkey Serum | 500µL |
| 1 x PBS | 4350µL |

**[Table 7]**

| 10% Donkey Serum/PBS (composition of 1mL) | |
|---|---|
| Component | Content |
| Donkey Serum | 100µL |
| 1 x PBS | 900µL |

**[Table 8]**

| 0.3% Triton X-100/PBS (composition of 10mL) | |
|---|---|
| Component | Content |
| 10% Triton X-100 | 300µL |
| 1 x PBS | 9700µL |

**[Table 9]**

| 0.2% Triton X-100/PBS (composition of 10mL) | |
|---|---|
| Component | Content |
| 10% Triton X-100 | 200µL |
| 1 x PBS | 9800µL |

The primary antibody reaction was performed with the primary antibody reaction solutions shown in the following Table 10.

**[Table 10]**

| Primary antibody reaction solutions | | |
|---|---|---|
| Experimental group shown in Fig.14 | Antibody used | Content ratio in reaction solution Antibody used:10% Donkey serum/PBS |
| VEGFR2-EGFP | Rat anti-GFP antibody (NACALAI TESQUE, INC (No.04404-84) | 1:1000 |
| Neuro filament | Chicken-Neuro Filament (Novus NB300-217) | 1:1000 |

The secondary antibody reaction was performed with the secondary antibody reaction solutions shown in Table 11 below.

**[Table 11]**

| Secondary antibody reaction solutions | | |
|---|---|---|
| Experimental group shown in Fig.14 | Antibody used | Content ratio in reaction solution Antibody used:10% Donkey serum/PBS |
| VEGFR2-EGFP | Donkey anti-Rat IgG (H+L) Highly Cross-Adsorbed Secondary Antibody, Alexa Fluor 488 Invitrogen #A-21208 | 1:500 |
| Neuro filament | Anti-chicken IgY (IgG) (H+L), Donkey, Alexa Fluor^{™} 647 labeling, Affinity purification, Jackson Immuno Research Laboratories #703-605-155 | 1:500 |

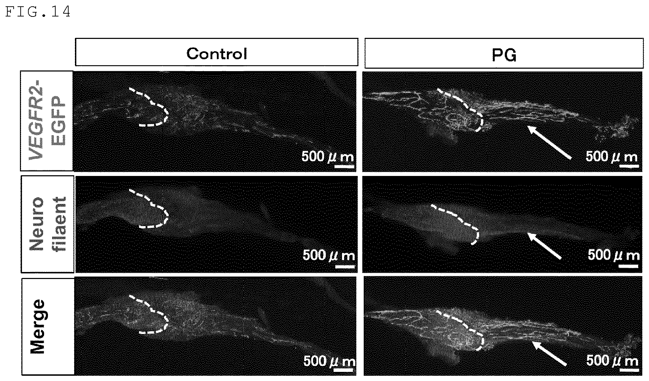

The results of this experiment 9 are shown in Fig.14. In Fig.14, "VEGFR2-EGFP" is a group of experiments to see if angiogenesis is taking place (confirmed by green expression of EGFP), and "Neuro filament" is a group of experiments to see if nerve regeneration is taking place (confirmed by red expression). "Merge" is a combination of "VEGFR2-EGFP" and "Neuro filament".

As shown in "VEGFR2-EGFP", compared to the group of control group treatment (labeled "Control" in Fig. 14), the PG treatment group (indicated as PG in Fig. 14) showed a marked green expression (arrow in Fig. 14). This expression indicates that angiogenesis is taking place. As shown in "Neuro filament", compared to the group of control group treatment, the PG-treated group showed a marked green expression (arrow in Fig. 14). This expression indicates that angiogenesis is taking place. As shown in "Merge," the areas of angiogenesis and nerve regeneration overlapped in the PG treatment group.

### [Experiment 10: Assessment of motor function using a sciatic nerve injury model]

Using a model mouse with an injured sciatic nerve (sciatic nerve transected), whether the administration of the unit containing a gel component (Matrigel) and proteoglycans improves motor function was evaluated by BBB scoring_{∘}

BBB scoring is one of the open field tests. BBB scoring is a method of evaluation that involves observing the behavior of subjects (in this experiment, the model mice treated with the following control group treatments, and model mice treated with the 10mg/mL PG group treatment), when they are released into a space of about 40 to 60 cm square. Table 12 shows the BBB scoring for this experiment 10.

**[Table 12]**

| BBB scoring table | |
|---|---|
| Score | Behavior |
| 0 | No observable hindlimb (HL) movement |
| 1 | Slight movement of one or two joints, usually the hip and/or knee |
| 2 | Extensive movement of one joint and slight movement of one other joint |
| 3 | Extensive movement of two joints of the HL |
| 4 | Slight movement of all three joints of the HL |
| 5 | Slight movement of two joints of the HL and extensive movement of one more joint |
| 6 | Extensive movement of two joints of the HL and slight movement of one more joint |
| 7 | Extensive movement of all three joints of the HL |
| 8 | Sweeping with no weight support using all the HL |
| 9 | Plantar placement of the paw with weight support in stance only (i.e., when stationary) |
| 10 | Occasional weight-supported plantar steps; no FL-HL coordination |
| 11 | Frequent to consistent weight-supported plantar steps and no FL-HL coordination |
| 12 | Frequent to consistent weight-supported plantar steps and occasional FL-HL coordination |
| 13 | Frequent to consistent weight-supported plantar steps and frequent FL-HL coordination |
| 14 | Consistent weight-supported plantar steps, consistent FL-HL coordination, and predominant paw position during locomotion is rotated |
| 15 | Consistent plantar stepping, consistent FL-HL coordination, and no toe clearance or occasional toe clearance during forward limb advancement; predominant paw position is parallel to the body at initial contact |
| 16 | Consistent plantar stepping, consistent FL-HL coordination, and toe clearance occurs frequently during forward limb advancement; predominant paw position is parallel to the body at initial contact and rotated at lift off |
| 17 | Consistent plantar stepping, consistent FL-HL coordination, and toe clearance occurs frequently during forward limb advancement; predominant paw position is parallel to the body at initial contact and lift off |
| 18 | Consistent plantar stepping, consistent FL-HL coordination, and toe clearance occurs consistently during forward limb advancement; predominant paw position is parallel to the body at initial contact and rotated at lift off |
| 19 | 18 points plus the condition of the tail, which is down part or all of the time |
| 20 | 18 points plus the condition of the tail, which is consistently up, but trunk instability |
| 21 | 20 points plus consistent trunk stability |

Experimental methods are described. First, 6-week-old female vascular reporter mice (Flt1-tdsRed BAC transgenic mice) were prepared. The sciatic nerves toward the tail of the two mice (sciatic nerves in the right and left limbs) were severed to create the model mice.

One model mouse (n=1) was given a control group treatment at the amputated site. The other model mouse (n=1) received 10mg/mL PG group treatment at the transected site.

Here, the following definitions are used.
- PG group treatment: Treatment in which a unit containing a proteoglycan (final concentration of 10mg/mL) in Matrigel is placed on the sciatic nerve. (the site where the sciatic nerve was injured (severed)).
- Control group treatment: Treatment in which Matrigel is placed on the sciatic nerve (the site where the sciatic nerve was injured (severed)).
- Proteoglycan (FUJIFILM Wako Pure Chemical Corporation (Product Code: 162-22131, 168-22133)): Proteoglycan, derived from salmon nasal cartilage.
- Matrigel (Corning, Product Number: 356231): Corning^{™} Matrigel Basement Membrane Matrix Growth Factor Reduced Phenol red free.

Immediately after the treatment, the muscle layer and skin of the site of the treated model mice were sutured. After the suture, the model mice were reared as usual and BBB scoring (counting of scores based on Table 12) was performed. The results are shown in Table 13. In Table 13, "2 hours" refers to the score at 2 hours after the suture, "1 day" refers to the score at 1 day after the suture, and "8 days" refers to the number of points obtained 8 days after the suture, "17 days" refers to the number of points obtained 17 days after the suture.

**[Table 13]**

| Results of BBB scoring | | |
|---|---|---|
| | Treatment of control group | Treatment of PG group |
| 2 hours | 2 | 2 |
| 1 day | 2 | 2 |
| 8 days | 16 | 16 |
| 17 days | 16 | 18 |

As shown in Table 13, compared to the group of Control group treatment, the group of PG group treatment showed an improvement in motor function (the hindlimb treated in this study always showed a vigorous kicking motion with the toes, and the direction of the toes was parallel to the trunk both at ground contact and at release) at "17 days".

### [Experiment 11: Evaluation of angiogenic potential in a sciatic nerve injury model]

Using a model mouse with an injured sciatic nerve (sciatic nerve transected model mouse), *in vivo* imaging was used to evaluate the regenerative potential of blood vessels by administration of the unit containing gel components (Matrigel) and proteoglycans, and the like. Evaluation using the imaging was performed using the IVIS Imaging System (PerkinElmer) including equipment.

Experimental methods are described. First, 9-week-female vascular reporter mice (Flt1-tdsRed BAC transgenic mice) were prepared. The sciatic nerves toward the tail of the mice (sciatic nerves in the right and left limbs) were severed to create the model mice (n=1). The left sciatic nerve of the model mice was treated with the 10mg/mL PG group, and the right sciatic nerve of the model mice was treated with the PG+SU4312 group.

Here, the following definitions are used.
- PG group treatment: Treatment in which the unit containing a proteoglycan (final concentration of 10mg/mL) in Matrigel is placed on the sciatic nerve (the site where the sciatic nerve was injured (severed)).
- PG+SU4312 group treatment: Treatment in which a unit containing a proteoglycan (final concentration of 10mg/mL) and SU4312 (final concentration of 10µM) in Matrigel is placed on the sciatic nerve (the site where the sciatic nerve was injured (severed)).
- Proteoglycan (FUJIFILM Wako Pure Chemical Corporation (Product Code: 162-22131, 168-22133)): Proteoglycan, derived from salmon nasal cartilage.
- Matrigel (Corning, Product Number: 356231): Corning^{™} Matrigel Basement Membrane Matrix Growth Factor Reduced Phenol red free.
- SU4312 (S8567, Sigma-Aldrich): Inhibitor of VEGF receptor

Immediately after the treatment, the muscle layer and skin of the site of the treated model mice were sutured. After the suture, the alfalfa-free fluorescence imaging feed (5V5M, PicoLab^{R} SelectMouse 50IF/9F) was also used for 7 days of normal rearing of the model mice.

On the seventh day of the breeding, the AngioSense^{™} 750EX (PerkinElmer, NeV10011EX) was injected into the tail vein of the model mice (dose of 2nmol/100pL of AngioSense 750 EX per mouse). Normal rearing was continued for a certain period of time after the injection. The IVIS of the model mice were photographed 24 hours after the injection.

The conditions for such photography are shown below.
- Exposure: 5.00 sec.
- Bining: Medium
- Fstop: 2
- Excitation: 640
- Emission: Cy5.5

Immediately before the imaging, the model mice were given general anesthesia, and after the anesthesia, body hair was removed from a predetermined area of the model mice. After the hairs were removed, the images were taken. The general anesthesia was performed by placing the model mice in a suction anesthesia box with anesthesia circulating for a certain period of time.
The removal was performed in the vicinity of the site where the above-mentioned treatment (PG group treatment or Control group treatment).

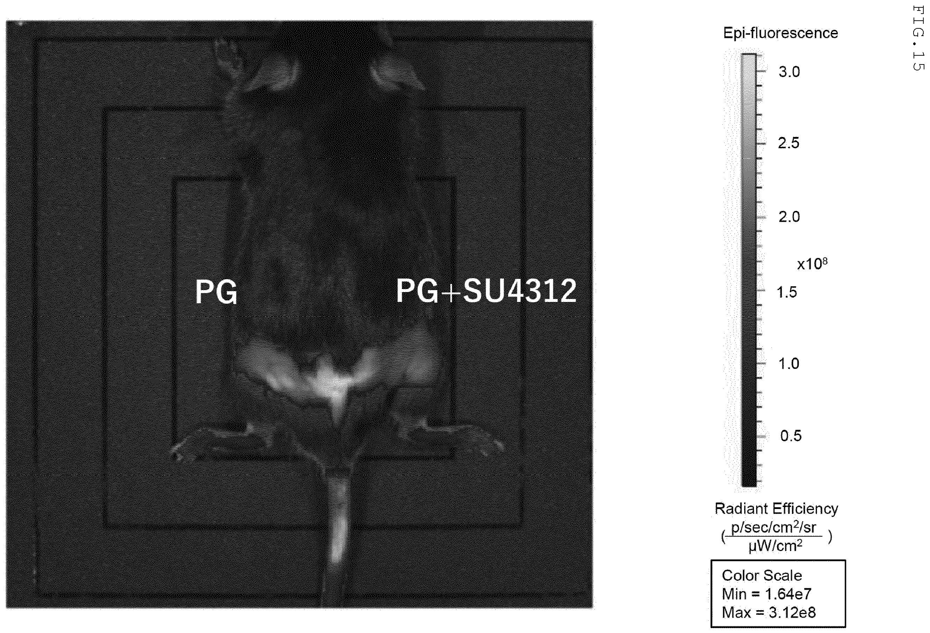

The results of the imaging are shown in Fig.15. On the right side of Fig.15, degree of fluorescence (Epi-fluorescence described in Fig.15) is shown. The degree of fluorescence is expressed in terms of the radiant efficiency (equation in Fig. 15, the radiant efficiency). The yellow color indicates more angiogenesis *in vivo* in mice. PG+SU4312 treatment group (labeled PG+SU4312 in Fig.15), shows lower yellow expression compared to the PG group (labeled PG in Fig.15).

In the group shown in Fig.15, Total Radiant Efficiency ([p/s]/[µW/cm²]) and Average Radiant Efficiency ([p/s/cm²/sr]/[µW/cm²) were quantified in the area indicated by the rectangle in Fig.15. The results are shown in Table 14. The "PG", and "PG+SU4312" in the table indicate the groups shown in Fig.15. The quantitative results also show that, the PG+SU4312 treatment group showed a lower amount of yellow color compared to the group of PG group treatment. The results of the PG+SU4312 treatment group suggest that further angiogenesis may occur if there is additional VEGF in the PG group.

**[Table 14]**

| Quantitative results | | |
|---|---|---|
| | PG | PG + SU4312 |
| Total Radiant Efficiency ([p/s]/[µW/cm²]) | 9.87 x 10⁸ | 7.79 x 10⁸ |
| Average Radiant Efficiency ([p/s/cm²/sr]/[µW/cm²]) | 11.9 x 10⁷ | 8.55 x 10⁷ |

### [Experiment 12: Confirmation of aggrecan expression, etc. in mouse brain tissue]

In order to understand how aggrecan (proteoglycan) functions in the process of angiogenesis in brain, we checked its expression. Aggrecan is a major structural proteoglycan present in the extracellular matrix of cartilage tissue. Its molecular weight is over 2,500 kDa, and it is composed of 100 to 150 glycosaminoglycan (GAG)chains attached to the core protein. Versican is a large proteoglycan distributed in a wide range of tissues. It has a complex structure with many N- and O-linked sugar chains. The core protein of versican has hyaluronic acid binding ability and is responsible for the organization and maintenance of the extracellular matrix.

The experimental methods for this experiment 12 are described. Brains from 15.5-day old mice , 17.5-day old mice, 1.5-month-old mice and 14.5-month-old mice were collected. The mice used in this experiment 10 were male vascular reporter mice (Flt1-tdsRed BAG transgenic mouse).

The brain samples were washed with PBS for 5 minutes. After the wash, cells were fixed in 4% paraformaldehyde for 1 hour. After fixation, the brains were embedded in a 5% agarose gel. The encapsulated brains were cut using a vibratome, and 150-um-thick sections were prepared. The sections were blocked in PBS solution containing 0.3% Triton and 10% Donkey serum for one day and night. After the blocking, the sections were subjected to a primary antibody reaction in a reaction solution containing the prescribed antibody (primary antibody reaction solution) for one day and night. After the primary antibody reaction, the sections were washed with PBS, and the washed sections were subjected to a secondary antibody reaction in a secondary antibody reaction solution for one day and night. After the secondary antibody reaction, the sections were washed with PBS, and the washed sections were sealed with the prescribed mounting agent.

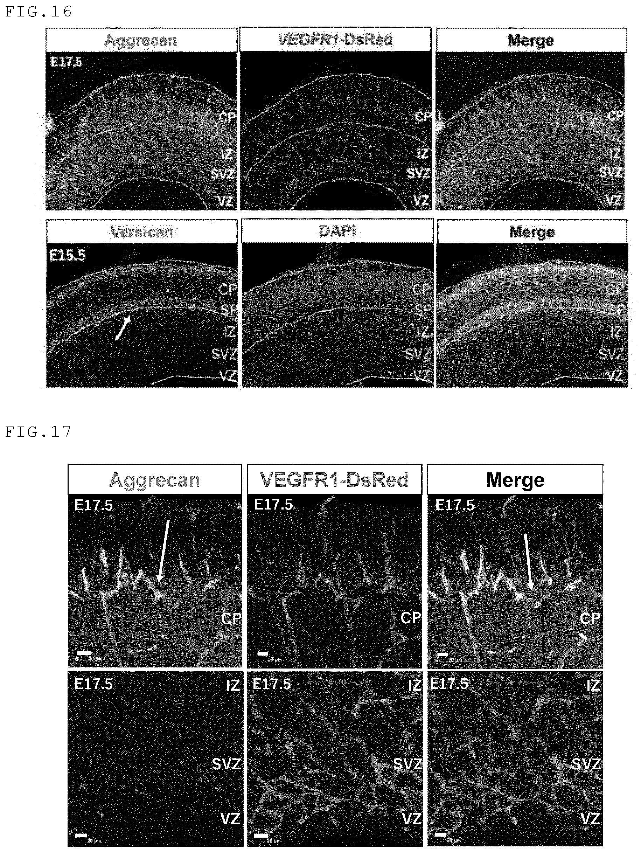

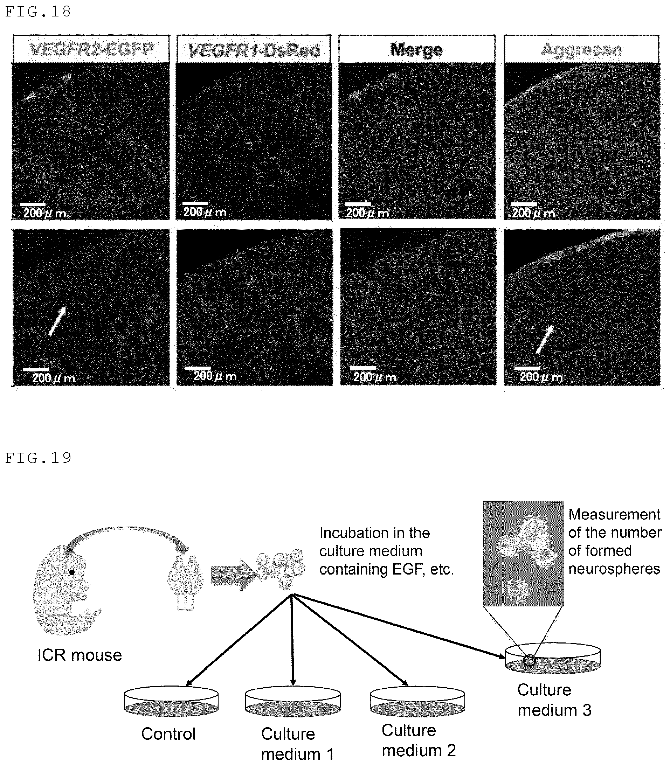

The sections after such inclusion were examined using a confocal microscope (Olympus Corporation: Confocal Laser Scanning Microscope FV3000) at magnifications of 10x (Fig.16), 40x (Fig.17), and 20x (Fig.18) to confirm the expression of aggrecan.

The results of this experiment 12 are shown in Figs.16 through 18. Fig.17 shows the arrowed region of the E17.5 group in Fig.16 at 4-fold magnified view. As shown in Fig.16, in the developing cortex, aggrecan is expressed in the vasculature and versican in the nervous system. As shown in Fig.17, aggrecan was expressed in capillaries near the cortical plate. As shown in Fig.18, comparing M1.5 and M14.5, a loss of aggrecan expression was observed with aging in M14.5.

In Figs.16 through 18, the following is marked.
- E15.5: Group of mouse brains at 15.5 days of fetal age.
- E17.5: Group of mouse brains at 17.5 days of fetal age.
- M1.5: Group of brains of 1.5-month-old mouse.
- M14.5: Group of brains of 14.5-month-old mouse.
- Aggrecan: experimental group immunostained for aggrecan by anti-aggrecan antibody
- VEGFR1-DSRed: Experimental group that showed the staining with DSRed (fluorescent protein) by immunostaining VEGFR1 with anti-VEGFR1 antibody.
- VEGFR2-EGFP: Experimental group that showed the staining with EGFP (Enhanced Green Fluorescent Protein) by immunostaining VEGFR2 by anti-VEGFR2 antibody.
- Versican: Experimental group of immunostained Versican by anti-Versican antibody.
- DAPI: DAPI-stained experimental group
- CP: Cortical plate
- SP: Subplate
- IZ: Intermediate zone
- SVZ: subventricular zone
- VZ: Ventricular zone

In this experiment 12, the primary antibody reaction solution shown in Table 15 below and the secondary antibody reaction solution shown in Table 16 below were used.

**[Table 15]**

| Primary antibody reaction solution | | |
|---|---|---|
| Experimental group shown in Figs.15-17 | Antibody used | Content ratio in reaction solution Antibody used:10% Donkey serum/PBS |
| VEGFR2-EGFP | Rat anti-GFP antibody (NACALAI | 1:1000 |
| | TESQUE, INC (#04404-84) | |
| Versican | Anti-Versican antibody, a.a. 1360-1439 of mouse versican Merck #AB1033 (Rabbit anti-Versican antibody) | 1:100 |

**[Table 16]**

| Secondary antibody reaction solution | | |
|---|---|---|
| Experimental group shown in Fig.14 | Antibody used | Content ratio in reaction solution Antibody used:10% Donkey serum/PBS |
| VEGFR2-EGFP and Versican | Donkey anti-Rat IgG (H+L) Highly Cross-Adsorbed Secondary Antibody, Alexa Fluor 488 Invitrogen #A-21208 | 1:500 |
| Versican | Donkey anti-Rabbit IgG (H+L) Highly Cross-Adsorbed Secondary Antibody, Alexa Fluor 594 Invitrogen #A-21207 | 1:500 |

### [Experiment 13: Measurement of the Number of Formed Neurospheres (NS) and Observation of NS]

To confirm that the cells cultured in the presence of FGF-2, EGF and proteoglycans are neural stem cells, the number of formed neurospheres (NS) were measured and the morphology of NS was observed.

### (Procedures for Culturing Neural Stem Cells)

The procedure (experimental procedure) for culturing neural stem cells in Experiment 13 is described below. A schematic diagram of this experimental procedure is shown in FIG.19. A female mouse on the 14th day of gestation was prepared using ICR mice manufactured by Japan SLC Co. The fetus mouse present in the body of the female mouse was then removed, to prepare cells dispersed from the cerebral cortex of the fetus mouse. In this dispersion, 0.05 w/v% trypsin (prepared by diluting a product of FUJIFILM Wako Pure Chemical Corporation 0.25w/v% Trypsin-1mmol/L EDTA-4Na solution (containing phenol red) (product code: 201-16945, 209-16941))in PBS was used. The following treatment groups (control, culture medium 1, culture medium 2, and culture medium 3) were prepared using 240,000 cells of these dispersed cells. These cells were used 60,000 cells/each sample, respectively. The cells were inoculated into a 6-well plate (Thermo Fisher Scientific, Inc., CAT #140675) with the predetermined liquid culture medium. These cells were inoculated evenly 10,000 cells in each well of the 6-well plate with 2 ml of the predetermined liquid culture medium. After 7 days of culture, the formed cell aggregations in the 6-well plate of each treatment group were observed using a prescribed phase contrast microscope (magnification 20x). The cell aggregations with a radius of 50um or more were designated as neurospheres, and the number of neurospheres was measured. As a comparison to the observation after 7 days of culture, the observation on the first day of culture of each treatment group was also performed in the same way using a prescribed phase contrast microscope (magnification 20x).

The composition of the liquid culture medium for each group (control, culture medium 1, culture medium 2, and culture medium 3) is as follows. DMEM/HamF-12 (NACALAI TESQUE, INC., 08460-95) was used as the basic medium in all groups, and the composition of each component in 50ml is shown below.

### (Control)

- N2 500µl
- B27 1ml
- Heparin 50µl (final concentration: 2ng/ml)
- Antibiotics 100µl (Final concentration of penicillin 100U/ml, streptomycin 10µg/ml)
- bFGF 50µl (final concentration 10ng/ml)
- EGF 50µl (final concentration 10ng/ml)
- Proteoglycan no inclusion

### (Culture Medium 1)

- N2 500µl
- B27 1ml
- Heparin 50µl (final concentration: 2ng/ml)
- Antibiotics 100µl (Final concentration of penicillin 100 U/ml, streptomycin 10µg/ml)
- bFGF 50µl (final concentration 10ng/ml)
- EGF 50µl (final concentration 10ng/ml)
- Proteoglycan final concentration of 10µg/ml

### (Culture Medium 2)

- N2 500µl
- B27 1ml
- Heparin 50µl (final concentration: 2ng/ml)
- Antibiotics 100µl (Final concentration of penicillin 100U/ml, streptomycin 10µg/ml)
- bFGF 50µl (final concentration 10ng/ml)
- EGF 50µl (final concentration 10ng/ml)
- Proteoglycan final concentration of 100ug/ml

### (Culture Medium 3)

- N2 500µl
- B27 1ml
- Heparin 50µl (final concentration: 2ng/ml)
- Antibiotics 100µl (Final concentration of penicillin 100U/ml, streptomycin 10µg/ml)
- bFGF 50µl (final concentration 10ng/ml)
- EGF 50µl (final concentration 10ng/ml)
- Final proteoglycan concentration of 1000ug/ml

The ingredients contained in each of these groups (control, culture medium 1, culture medium 2, and culture medium 3) were as follows.
- N2: N-2 Supplement (100x) (gibco:17502-048)
- B27: B-27^{™} Plus Supplement Vitamin-A-free (50x) (gibco:A35828-01)
- Heparin: Heparin sodium (NACALAI TESQUE, INC. (product codes: 17513-96, 17513-41, 17513-54)
- Antibiotics: penicillin-streptomycin (WAKO: 168-23191)
- bFGF: bFGF (Funakoshi Co., Ltd.:100-18B)
- EGF: EGF (Funakoshi Co., Ltd.:315-09)
- Proteoglycan: Proteoglycan, derived from salmon nasal cartilage (FUJIFILM Wako Pure Chemical Corporation (product code: 162-22131, 168-22133))

### [Results of Measurement of the Number of Formed Neurospheres (NS) in Each Treatment Group]

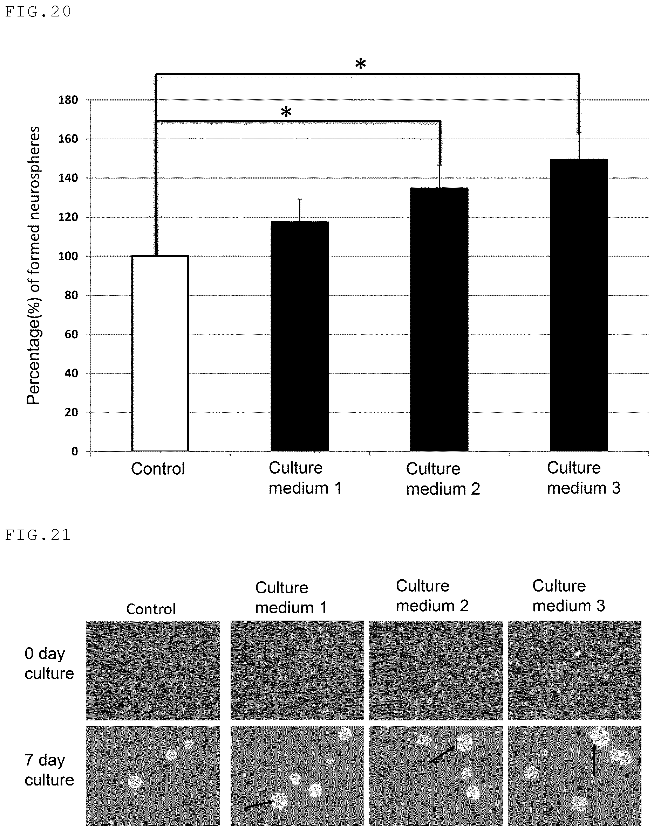

The results of this measurement are shown in FIG.20. What is shown in this figure is the percentage (%) of NS formation when the number of NS formation in the control group is 100(%). Culture medium 1 is 117.317753376014%, culture medium 2 is
134.677950940218%, and culture medium 3 is 149.305508553623%. It was observed that the number of NS formation increased with the increase in proteoglycan content. For culture medium 2 and culture medium 3, it was confirmed that the number of NS formation was increased with statistically significance compared to the control.

### [Results of NS Observation]

The results of this observation are shown in FIG.21, where the observations after 7 days of culture ("7 day culture" in FIG.21) are also compared with those after 0 days of culture ("0 day culture" in FIG. 21). Compared to the control, many more NS were observed in culture media 1-3. Some NSs are indicated by arrows in FIG.21.

Therefore, it was found that much more neural stem cells proliferate by the inclusion of FGF-2, EGF and proteoglycans.

### [Experiment 14: Dual-Luciferase Assay (Confirmation of Transcriptional Activity of Hes-1)]

By checking the transcriptional activity of Hes-1, a protein (transcription factor) that regulates the maintenance of undifferentiated state of neural stem cells and their differentiation into astrocytes, we confirmed whether the neural stem cells described above (cells cultured in the presence of FGF-2, EGF, and proteoglycans) were maintained in their undifferentiated state.

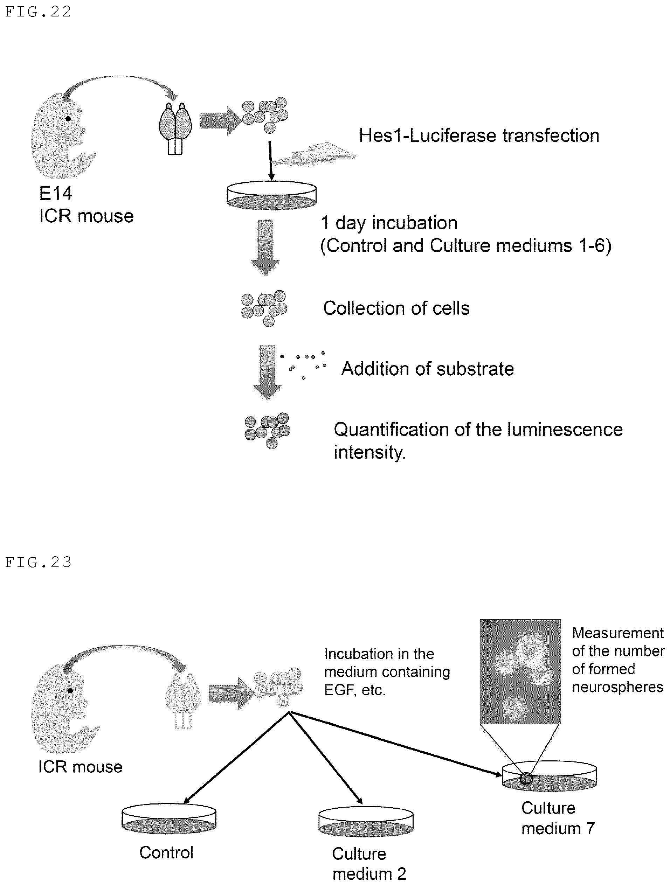

The procedure for this dual-luciferase assay is described below. First, neural stem cells were prepared. A schematic diagram of this experimental procedure is shown in FIG.22. We generated female mice on the 14th day of gestation using ICR mice manufactured by Japan SLC Co. The fetus mouse present in the body of the female mouse was then removed, to prepare cells dispersed from the cerebral cortex of the fetus mouse. In this dispersion, 0.05 w/v% trypsin (prepared by diluting a product of FUJIFILM Wako Pure Chemical Corporation 0.25 w/v% Trypsin-1mmol/L EDTA-4Na solution (containing phenol red) (product code: 201-16945, 209-16941))in PBS was used. The dispersed cells were divided into approximately 60,000 cells/each sample to prepare samples for the following seven treatment groups (control, culture medium 1, culture medium 2, culture medium 3, culture medium 4, culture medium 5, culture medium 6).

These samples were genetically transfected with Hes-1 cDNA and reporter gene (luciferase gene) using transfection reagent (Neon (registered trademark) Transfection System, Thermo Fisher Scientific). The Hes-1 cDNA and reporter gene (luciferase gene) were "pHes1(2.5 k)-luc (Plasmid #43806)" manufactured by Addgene. After this gene transfer, the cells were cultured in the liquid culture medium of each treatment group (control, culture medium 1, culture medium 2, culture medium 3, culture medium 4, culture medium 5, and culture medium 6) at 37°C. and 5% CO₂ for 1 day. After 1 day of culture, luciferase substrate (Thermo Fisher Scientific, Neon^{™} Transfection System) was added to each sample, and the expression of luciferase in each sample was observed using a detection system (Promega Corporation, GloMax^{™} Discover Microplate Reader).

The composition of the liquid culture medium for each dose group (control, culture medium 1, culture medium 2, culture medium 3, culture medium 4, culture medium 5, and culture medium 6) used in this dual-luciferase assay is as follows. The composition of the control, culture medium 1, culture medium 2, and culture medium 3 is the same as the liquid culture medium used in Experiment 1 above. DMEM/HamF-12 (NACALAI TESQUE, INC., 08460-95) was used as the basic medium in all dose groups, and the composition of each component in 50ml is shown below. In this culture, "Costar^{™} 24-well" from Corning Inc. was used.

### (Control)

- N2 500µl
- B27 1ml
- Heparin 50µl (final concentration 2ng/ml)
- Antibiotics 100µl (Final concentration of penicillin 100U/ml, streptomycin 10µg/ml)
- bFGF 50µl (final concentration 10ng/ml)
- EGF 50µl (final concentration 10ng/ml)
- No proteoglycan is included.

### (Culture Medium 1)

- N2 500µl
- B27 1ml
- Heparin 50µl (final concentration 2ng/ml)
- Antibiotics 100µl (Final concentration of penicillin 100U/ml, streptomycin 10µg/ml)
- bFGF 50µl (final concentration 10ng/ml)
- EGF 50µl (final concentration 10ng/ml)
- Final concentration of proteoglycan 10µg/ml

### (Culture Medium 2)

- N2 500µl
- B27 1ml
- Heparin 50µl (final concentration: 2 ng/ml)
- Antibiotics 100µl (Final concentration of penicillin 100 U/ml, streptomycin 10µg/ml)
- bFGF 50µl (final concentration 10ng/ml)
- EGF 50µl (final concentration 10ng/ml)
- Final proteoglycan concentration of 100ug/ml

### (Culture Medium 3)

- N2 500µl
- B27 1ml
- Heparin 50µl (final concentration: 2ng/ml)
- Antibiotics 100µl (Final concentration of penicillin 100 U/ml, streptomycin 10µg/ml)
- bFGF 50µl (final concentration 10ng/ml)
- EGF 50µl (final concentration 10ng/ml)
- Final proteoglycan concentration of 1000ug/ml

### (Culture Medium 4)

- N2 500µl
- B27 1ml
- Heparin 50µl (final concentration: 2ng/ml)
- Antibiotics 100µl (Final concentration of penicillin 100 U/ml, streptomycin 10µg/ml)
- No bFGF inclusion
- EGF 50µl (final concentration 10ng/ml)
- Final proteoglycan concentration of 1000ug/ml

### (Culture Medium 5)

- N2 500µl
- B27 1ml
- Heparin 50µl (final concentration: 2ng/ml)
- Antibiotics 100µl (Final concentration of penicillin 100U/ml, streptomycin 10g/ml)
- bFGF 50µl (final concentration 10ng/ml)
- No EGF inclusion
- Final proteoglycan concentration of 1000ug/ml

### (Culture Medium 6)

- N2 500µl
- B27 1ml
- Heparin 50µl (final concentration: 2ng/ml)
- Antibiotics 100µl (Final concentration of penicillin 100 U/ml, streptomycin 10µg/ml)
- bFGF 150µl (final concentration 30ng/ml)
- EGF 50µl (final concentration 10ng/ml)
- No proteoglycan inclusion

The ingredients contained in each of these treatment groups (control, culture medium 1, culture medium 2, culture medium 3, culture medium 4, culture mediums and culture medium6) were as follows.
- N2: N-2 Supplement (100x) (Gibco: 17502-048)
- B27: B-27^{™} Plus Supplement containing no vitamin A (50x) (gibco: A35828-01)
- Heparin: Heparin sodium (NACALAI TESQUE, INC. (product codes: 17513-96, 17513-41, 17513-54))
- Antibiotics: penicillin-streptomycin (WAKO: 168-23191)
- bFGF: bFGF (Funakoshi Co., Ltd.:100-18B)
- EGF: EGF (Funakoshi Co., Ltd.:315-09)
- Proteoglycan: Proteoglycan, derived from salmon nasal cartilage (FUJIFILM Wako Pure Chemicals Company (product codes: 162-22131, 168-22133))

The observation of the expression level of luciferase (transcriptional activity of Hes-1) in each well is described below. When the expression level of the control group was 100, the expression level of the group in culture medium 1 was 123.83, the expression level of the group in culture medium 2 was 131.24, the expression level of the group in culture medium 3 was 159.51, the expression level of the group in culture medium 4 was 113.85, the expression level of the group in culture medium 5 was 119.67, and the expression level of the group in culture medium 6 was 131.55. The higher the expression level of luciferase, the higher the transcriptional activity of Hes-1, and the more undifferentiated neural stem cells are maintained. Culture medium 6 is considered as a positive control, which maintains the undifferentiated potential of neural stem cells. However, compared to culture medium 6 (which does not contain proteoglycans but has 3-fold higher bFGF content compared to the control), culture medium 1, culture medium 2, and culture medium 3 groups (FGF-2, EGF and proteoglycans) maintained the undifferentiated nature of neural stem cells.

### [Experiment 15: Measurement of the Number of NS Formation]

In a culture medium containing GAGs instead of proteoglycans, we checked whether cells cultured in this culture medium could form NSs. In this experiment, GAGs, unlike proteoglycans, do not have a core protein bound to them.

The procedure for this experiment 15 is described below. A schematic diagram of this experimental procedure is shown in FIG.23. A female mouse on the 14th day of gestation was created using ICR mice manufactured by Japan SLC Co. The mouse fetus present in the body of the mouse was then removed, and cells dispersed from the cerebral cortex of the mouse fetus were prepared. In this dispersion, 0.05 w/v% trypsin (prepared by diluting a product of FUJIFILM Wako Pure Chemical Corporation 0.25 w/v% Trypsin-1mmol/L EDTA-4Na solution (containing phenol red) (product code: 201-16945, 209-16941))in PBS was used. The following treatment groups (control, culture medium 2, culture medium 7) were prepared using 180,000 cells of these dispersed cells. These cells were used separately, 60,000 cells/each sample. The cells were inoculated into a 6-well plate (Thermo Fisher Scientific, Inc., CAT #140675) in which the prescribed liquid culture medium was present. 2ml of the prescribed liquid culture medium was inoculated evenly into each well of the 6-well plate so that 10,000 cells of these cells were present per well. After 6 days of culture, the formed cell masses in the 6-well plate of each treatment group were observed under a prescribed phase contrast microscope (magnification 20x). After 6 days of culture, the cell masses formed in the 6-well plates of each treatment group were observed under a phase contrast microscope (magnification: 20x), and the cell masses formed with a radius of 50um or greater were defined as neurospheres, and the number of these neurospheres was measured. As a comparison to the observation after 6 days of culture, the observation on the first day of culture of each treatment group was also performed in the same way using a prescribed phase contrast microscope (magnification 20x).

The composition of the liquid culture medium for each treatment group (control, culture medium 2, culture medium 7) is as follows. The composition of the control and culture medium 2 is the same as the liquid culture medium used in Experiment 13 above. DMEM/HamF-12 (NACALAI TESQUE, INC., 08460-95) was used as the basic culture medium in all dose groups, and the composition of each component in 50ml is shown below.

### (Control)

N2 500µl
B27 1ml
Heparin 50µl (final concentration: 2ng/ml)
Antibiotics 100µl (Final concentration of penicillin 100 U/ml, streptomycin 10µg/ml)
bFGF 50µl (final concentration 10ng/ml)
EGF 50µl (final concentration 10ng/ml)
No proteoglycan inclusion

### (Culture Medium 2)

N2 500µl
B27 1ml
Heparin 50µl (final concentration: 2ng/ml)
Antibiotics 100µl (Final concentration of penicillin 100 U/ml, streptomycin 10µg/ml)
bFGF 50µl (final concentration 10ng/ml)
EGF 50µl (final concentration 10ng/ml)
Final proteoglycan concentration of 100ug/ml

### (Culture Medium 7)

N2 500µl
B27 1ml
Heparin 50µl (final concentration: 2ng/ml)
Antibiotics 100µl (Final concentration of penicillin 100 U/ml, streptomycin 10µg/ml)
bFGF 50µl (final concentration 10ng/ml)
EGF 50µl (final concentration 10ng/ml)
Final GAG concentrations of 100ug/ml

The ingredients contained in each of these dose groups (control, culture medium 2, culture medium 7) were as follows.
- N2: N-2 Supplement (100x) (gibco:17502-048)
- B27: B-27^{™} Plus Supplement containing no vitamin A (50x) (gibco: A35828-01)
- Heparin: Heparin sodium (NACALAI TESQUE, INC. (product codes: 17513-96, 17513-41, 17513-54))
- Antibiotics: penicillin-streptomycin (WAKO: 168-23191)
- bFGF: bFGF (Funakoshi Co., Ltd.:100-18B)
- EGF: EGF (Funakoshi Co., Ltd.:315-09)
- Proteoglycan: Proteoglycan, derived from salmon nasal cartilage (FUJIFILM Wako Pure Chemical Company (product codes: 162-22131, 168-22133)), from which the GAG used in Experiment 3 is purified to be only GAG by the conventional method.

The results of the measurement of the number of formed NSs is described below. The number of formed NSs in the control group was 100(%), and the percentage of NSs formed in the control group was 58.8%. The number of NSs formed in the culture medium 2 group was 118.7%, and that in the culture medium 7 group was 58.8%. It was suggested that the structure of proteoglycans (the conjugate structure of the core protein and the GAG structure) promotes the formation of NS.

### [Experiment 16: Confirmation of proteoglycan localization in neural stem cells]

In neural stem cells grown in medium containing proteoglycans and other substances, we checked where the proteoglycans were localized.

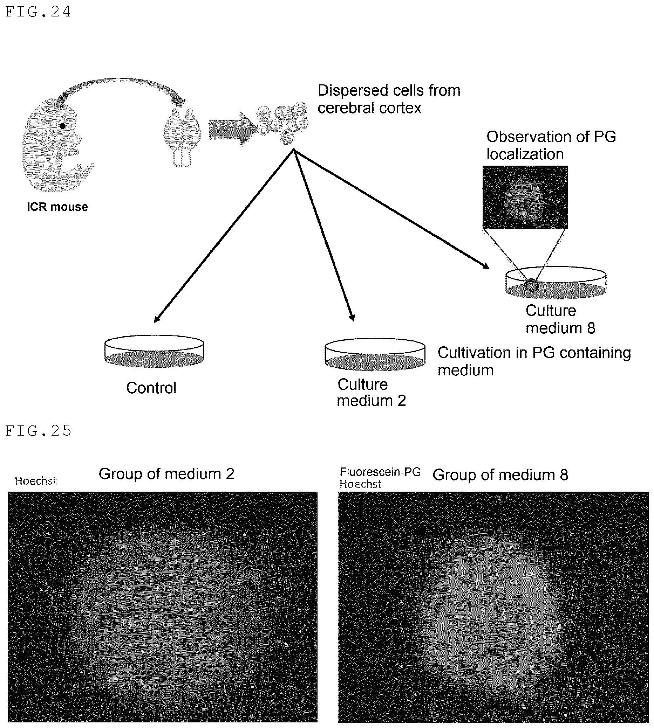

This confirmation procedure is described below. A schematic diagram of this experimental procedure is shown in Fig.24.

First, neural stem cells were prepared. A female mouse on the 14th day of gestation was prepared using ICR mice manufactured by Japan SLC Co. Then, the mouse fetus present in the body of the mouse was removed, and cells dispersed from the cerebral cortex of the mouse fetus were prepared. In this dispersion, 0.05 w/v% trypsin (prepared by diluting a product of FUJIFILM Wako Pure Chemical Corporation 0.25 w/v% Trypsin-1mmol/L EDTA-4Na solution (containing phenol red) (product code: 201-16945, 209-16941))in PBS was used. The following medium groups (medium 2 and medium 8) were prepared using 180,000 of these dispersed cells. These cells were used separately, 60,000 cells per sample. A 6-well plate (Thermo Fisher Scientific Corporation, CAT #140675) in which the prescribed liquid medium is present). 6-well plates were inoculated with 2ml of the prescribed liquid medium (the medium described in the relevant medium group) per well, and these cells were inoculated evenly into each well so that 10,000 cells were present. After this inoculation, the neural stem cells were cultured for 24 hours at 37°C, 5% CO₂ to grow the neural stem cells for observation. One hour before collecting the neural stem cells to be used for observation, Hoechst 33342 (final concentration of 10mg/mL, Hoechst^{™} 33342 imaging protocol (Thermo Fisher Scientific) was added and stained with the nuclei of neural stem cells. Observations were made using a fluorescence microscope (Olympus Corporation: Inverted Research Microscope IX81) at 20x magnification with the localization of proteoglycans in the formed cells in 6-well plates of each treatment group after 24 hours of culture was observed.

The composition of the liquid medium of each culture group (medium 2 and medium 8) used in this experiment 16 is as follows. Medium 2 has the same composition as the liquid medium used in Experiment 13 above. In all treatment groups, DMEM/Ham F-12 (NACALAI TESQUE, INC., 08460-95) was used as the basic medium, but in the following composition of each component in 50ml.

### (Medium 2)

- N2 500µl
- B27 1ml
- Heparin 50µl (final concentration 2ng/ml)
- Antibiotics 100µl (final concentration of penicillin 100U/ml, streptomycin 10µg/ml)
- bFGF 50µl (final concentration 10ng/ml)
- EGF 50µl (final concentration 10ng/ml)
- Proteoglycan Final concentration 100ug/ml

### (Medium 8)

- N2 500µl
- B27 1ml
- Heparin 50µl (final concentration 2ng/ml)
- Antibiotics 100µl (final concentration of penicillin 100U/ml, streptomycin 10µg/ml)
- bFGF 50µl (final concentration 10ng/ml)
- EGF 50µl (final concentration 10ng/ml)
- Fluorescently labeled proteoglycans Final concentration 100ug/ml

The components contained in each of these culture media groups (culture media 2 and 8) were as follows.
- N2: N-2 Supplement (100X) (gibco: 17502-048)
- B27: B-27^{™} Plus Supplement Vitamin A free (50X) gibco: A35828-01)
- Heparin: Heparin sodium (NACALAI TESQUE, INC. (Product Code: 17513-96, 17513-41, 17513-54)
- Antibiotic: Penicillin-Streptomycin (wako: 168-23191)
- 23191)
- bFGF: bFGF (Funakoshi Co., Ltd.:100-18B)
- EGF: EGF (FUNAKOSHI CO., LTD.:315-09)
- Proteoglycan: Proteoglycan, derived from salmon nasal cartilage (FUJIFILM Wako Pure Chemical Corporation (Product Code: 162-22) 131, 168-22 (133))

The "fluorescently labeled proteoglycans" in medium 8 were prepared as follows: ATTO 488 NHS ester (Sigma-Aldrich) and proteoglycans were combined with the manufacturer's recommended method (product: ATTO 488 NHS ester (Sigma-Aldrich)) for 2 hours at room temperature. After this reaction, the fluorescently labeled proteoglycans were purified using Zebra Spin Desalting Column (Thermo Fisher Scientific) and recovered. The recovered fluorescently labeled proteoglycans were used in this experiment 16.

The results of the observations are shown in Fig.25. Fig.25 shows the observation results of fluorescence microscopy of the group of medium 2 (medium containing non-fluorescently labeled proteoglycans unlike the group of medium 8) and the group of medium 8 (medium containing fluorescently labeled proteoglycans unlike the group of medium 2). In Fig.25, "Hoechst" indicates that the nuclei of neural stem cells were labeled by Hoechst staining (Hoechst^{™} 33342 imaging protocol (Thermo Fisher Scientific)) and "Fluorescein-PG" indicates that localized proteoglycans were labeled by fluorescence staining. In medium 8 group, fluorescently labeled proteoglycans were confirmed to be localized in the pericellular region of the neural stem cells. This confirmatory result suggests that proteoglycans added to the culture medium of neural stem cells localize to the pericellular area of neural stem cells and may be involved in the construction of the pericellular milieu (PCM).

### [Experiment 17: Confirmation of proteoglycan localization in neural stem cells by confocal microscopy.

The location of proteoglycans in neural stem cells grown in medium containing proteoglycans and other substances was also confirmed using confocal microscopy.

The procedure for this confirmation is described below. First, neural stem cells were prepared. A female mouse on the 14th day of gestation was prepared using ICR mice manufactured by Japan SLC Co. Then, the mouse fetus present in the body of the mouse was removed, and cells dispersed from the cerebral cortex of the mouse fetus were prepared. In this dispersion, 0.05 w/v% trypsin (prepared by diluting a product of FUJIFILM Wako Pure Chemical Corporation 0.25 w/v% Trypsin-1mmol/L EDTA-4Na solution (containing phenol red) (product code: 201-16945, 209-16941))in PBS was used. The following medium group (medium 9) was prepared using 180,000 of these dispersed cells. These cells were used separately, 60,000 cells/each sample. The cells were inoculated into a 6-well plate (Thermo Fisher Scientific, Inc., CAT #140675) in which the prescribed liquid culture medium was present. 2ml of the prescribed liquid culture medium was inoculated evenly into each well of the 6-well plate so that 10,000 cells of these cells were present per well. After the inoculation, the cells were cultured at 37°C, 5% CO₂ for 24 hours to allow the neural stem cells to grow and to prepare them for observation. The neural stem cells used for observation were prepared. One hour before collecting the neural stem cells to be used for observation, Hoechst 33342 was used to stain the nuclei of the neural stem cells (final concentration 10mg/mL, and Hoechst^{™} 33342 imaging protocol (Thermo Fisher Scientific)) and Cell mask (final concentration of 5mg/mL CellMask^{™} staining (Thermo Fisher Scientific) was added to stain and the cells were collected by centrifugation at 400g for 3 min, followed by fixation in 4% paraformaldehyde for 10 min. The cells thus prepared were washed with PBS and mounted on glass slides with an encapsulant to prepare observation materials. Observations were made using a confocal microscope (Olympus Corporation: Confocal Laser Scanning Microscope FV3000) at 40x magnification to compare the localization of nuclei of neural stem cells , plasma membrane of neural stem cells and proteoglycans.

The composition of the liquid medium for each culture group (medium 9) used in this experiment 17 is as follows. Note that DMEM/Ham F-12 (NACALAI TESQUE, INC. 08460-95) was used, and in the following, the composition of each component in 5ml is shown below.

### (Culture medium 9)

- N2 500µl
- B27 1ml
- Heparin 50µl (final concentration 2ng/ml)
- Antibiotics 100µl (final concentration of penicillin 100U/ml, streptomycin 10µg/ml)
- bFGF 50µl (final concentration 10ng/ml)
- EGF 50µl (final concentration 10ng/ml)
- Fluorescently labeled proteoglycans Final concentration 100ug/ml

The following ingredients were used in this medium 9.
- N2: N-2 Supplement (100X) (gibco: 17502-048)
- B27: B-27^{™} Plus Supplement Vitamin A free (50X) (gibco: A35828-01) A35828-01)
- Heparin: Heparin sodium (NACALAI TESQUE, INC. (Product code: 17513-96, 1)) Heparin: Heparin sodium (NACALAI TESQUE, INC. (Product Code: 17513-96, 1 Heparin: Heparin sodium (NACALAI TESQUE, INC. (product codes: 17513-96, 1 513-54)
- Antibiotic: Penicillin-Streptomycin (wako:168-23191)
- bFGF: bFGF (Funakoshi Co., Ltd.:100-18B)
- EGF: EGF (FUNAKOSHI CO., LTD.:315-09)
- Proteoglycan: Proteoglycan, derived from salmon nasal cartilage (FUJIFILM Wako Pure Chemical Corporation (Product Code: 162-22) 131, 168-22 (133))

The "fluorescently labeled proteoglycans" in medium 9 were prepared as follows: Atto 590 (Sigma-Aldrich, 70425) and proteoglycans were prepared by the method recommended by the manufacturer (product: Atto 590 (Sigma-Aldrich, 70425) for 2 hours at room temperature. After this reaction, Zeba Spin Desalting Column (Thermo Fisher Scientific) was used for purification, and the fluorescently labeled proteoglycans were recovered. The recovered fluorescently labeled proteoglycans were used in this experiment 5.

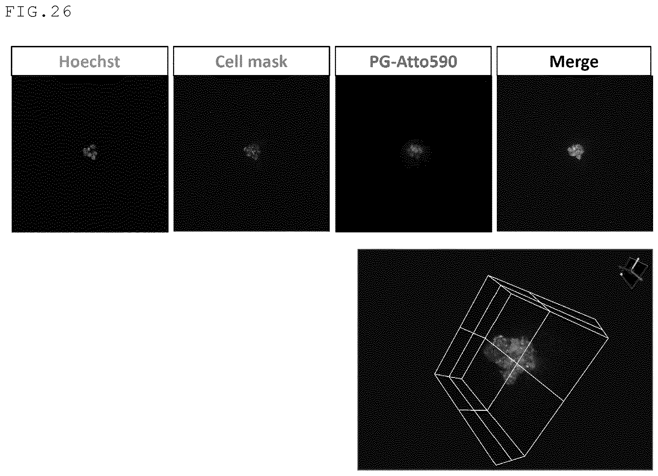

The results of the observation are shown in Fig.26. "Hoechst" indicates that the nuclei of the neural stem cells were labeled by Hoechst staining, and "Cell mask" indicates that the cell membrane of neural stem cells was labeled by CellMask^{™} staining, and "PG-Atto59" indicates that the proteoglycans were labeled, and "Merge" indicates that the labeling of nuclei, the labeling of cell membranes, and the labeling of proteoglycans were combined. The confirmatory results shown in Fig.26 indicate that, similar to the results shown in Fig.25, proteoglycans added to the culture medium of neural stem cells localize to the pericellular region of the neural stem cells, suggesting that proteoglycans added to the culture medium of neural stem cells may be involved in the construction of the pericellular environment (PCM).

While embodiments of the present invention (including examples) have been described above with reference to the drawings, the specific configuration of the present invention is not limited thereto, and even if there is a design change or the like within a range not departing from the gist of the present invention, it is included in the present invention.

## Claims

1. A unit for use in promoting angiogenesis and/or nerve regeneration, the unit comprising a gel component and a proteoglycan.

2. The unit according to claim 1, comprising VEGF.

3. The unit according to claim 1 or 2, comprising FGF-2.

4. The unit according to any one of claims 1 to 3, comprising EGF.

5. The unit according to any one of claims 1 to 4, further comprising cells for promoting angiogenesis.

6. The unit according to any one of claims 2 to 5, wherein a liquid medium comprising VEGF and the cells for promoting angiogenesis are stacked on top of the gel component.

7. The unit according to any one of claims 1 to 6, wherein a proteoglycan content is 0.1µg/ml or more.

8. The unit according to any one of claims 2 to 7, wherein the proteoglycan is contained in a liquid medium including VEGF.

9. The unit according to any one of claims 1 to 8, wherein the proteoglycan is contained in a gel component.
